# EUROPEAN PATENT APPLICATION

(11) **EP 2 075 255 A1**
(43) Date of publication of application: **01.07.2009**
(21) Application number: 09002470.4
(22) Date of filing: 07.03.2001
(51) Int. Cl.: C07K 14/50

(54) **Human FGF-23 gene and gene expression products**

(30) Priority: 08.03.2000 US 187854 P; 18.09.2000 US 233368 P; 05.12.2000 US 251650 P
(62) Divisional of application: 01922308.0
(71) Applicant: Novartis Vaccines and Diagnostics, Inc., Emeryville, CA 94608 (US); Kyoto University, Kyoto 606-8501 (JP)
(72) Inventor: Itoh, Nobuyuki, Kyoto 606-8501 (JP); Kavanaugh, Michael W., Mill Valley California 94941 (US)
(74) Representative: Bentham, Andrew

(57) **Abstract**

This invention relates to human fibroblast growth factor (hFGF-23), to cleavage products of hFGF-23, and to variants thereof and to polynucleotides encoding FGF-23. This invention also relates to diagnostic and therapeutic agents related to the polynucleotides and proteins, including probes and antibodies, and to methods of treating skin, brain and placental disease and disorders, and methods of treating conditions related to thymic function. The invention also relates to mouse fibroblast growth factor (mFGF-23), and to variants thereof and polynucleotides encoding mFGF-23.

## Description

### TECHNICAL FIELD

The present invention relates to nucleic acid sequences encoding a member of the fibroblast growth factor (FGF) family, and to polypeptides encoded by the nucleic acid sequence.

### BACKGROUND OF THE INVENTION

The prototypic fibroblast growth factors (FGFs), FGF-1 and FGF-2, were originally isolated from brain and pituitary as mitogens for fibroblasts. However, FGF-1 and FGF-2 are widely expressed in developing and adult tissues, and are polypeptides with multiple biological activities including angiogenesis, mitogenesis, cellular differentiation and repair of tissue injury (Baird, A. et al., Cancer Cells 3:239-243 (1991); Burgess, W.H. et al., Annu. Rev. Biochem. 58:575-606 (1989). According to the published literature, the FGF family now consists of at least nineteen members, FGF-1 to FGF-19. FGF-3 was identified to be a common target for activation by the mouse mammary tumor virus (Dickson et al., Ann. N.Y. Acad. Sci. 638:18-26 (1991); FGF-4 to FGF-6 were identified as oncogene products (Yoshida et al., Ann. NY Acad. Sci. 638:27-37 (1991); Goldfarb et al., Ann. NY Acad. Sci 638:38-52 (1991); Coulier et al., Ann. NY Acad. Sci. 638:53-61 (1991)). FGF-10 was identified from rat lung by homology-based polymerase chain reaction (PCR) (Yamasaki et al., J. Biol. Chem. 271:15918-15921 (1996)). FGF-11 to FGF-14 (FGF homologous factors (FHFs) 1 to 4) were identified from human retina by a combination of random cDNA sequencing, data base searches and homology-based PCR (Smallwood et al., Proc. Natl. Acad. Sci. USA 93:9850-9857 (1996)). FGF-15 was identified as a downstream target of a chimeric homeodomain oncoprotein (McWhirter et al., Development 124:3221-3232 (1997)). FGF-16, FGF-17, and FGF-18 were identified from rat heart and embryos by homology-based PCR, respectively (Miyake et al., Biochem. Biophys. Res. Commun. 243:148-152 (1998); Hoshikawa et al., Biochem. Biophys. Res. Commun. 244:187-191 (1998); Ohbayashi et al., J. Biol. Chem. 273:18161-18164 (1998)). Recently, FGF-19 was identified from human fetal brain by data base search (Nishimura et al., Biochim. Biophys. Acta 1444:148-151 (1999)). They have a conserved∼120-amino acid residue core with ∼30 to 60% amino acid identity. These FGFs also appear to play important roles in both developing and adult tissues. Thus, there is a need in the art for additional FGF molecules having functions and activities that differ from the known FGFs and for FGF molecules specifically expressed in tissues implicated in human disease.

### SUMMARY OF THE INVENTION

The present invention provides a composition comprising an isolated polynucleotide selected from the group consisting of:
(a) a polynucleotide comprising at least eight contiguous nucleotides of SEQ ID NO:1 or 3;
(b) a polynucleotide having at least 80% homology to the polynucleotide of (a); and
(c) a polynucleotide encoding a protein expressed by a polynucleotide having the sequence of SEQ ID NO:1 or 3.

The invention further provides for the use of the isolated polynucleotides or fragments thereof as diagnostic probes or as primers.

The present invention also provides a composition comprising a polypeptide, wherein said polypeptide is selected from the group consisting of:
(a) a polypeptide comprising at least 6 contiguous amino acids encoded by SEQ ID NO:1 or 3;
(b) a polypeptide encoded by a polynucleotide comprising SEQ ID NO:1 or 3; and
(c) a variant of the polypeptide of SEQ ID NO:2 or 4.

In certain preferred embodiments of the invention, the polynucleotide is operably linked to an expression control sequence. The invention further provides a host cell, including bacterial, yeast, insect and mammalian cells, transformed with the polynucleotide sequence. The invention also provides full-length cDNA and full-length polynucleotides corresponding to SEQ ID NO:1 or 3.

In other preferred embodiments, the FGF-23 is a noncleavable mutant, wherein a cleavage site is mutated. In specific embodiments, the amino acids at positions 176 and 179 are substituted or deleted. In other embodiments, the amino acids at positions 176 and 179 are not changed, but one or more amino acids near position 176 and/or 179 is mutated, also resulting in a non-cleavable form of FGF-23.

Protein and polypeptide compositions of the invention may further comprise a pharmaceutically acceptable carrier. Compositions comprising an antibody that specifically reacts with such protein or polypeptide are also provided by the present invention.

The invention also provides for the production of large amounts of otherwise minor cell populations of cells to be used for generation of cDNA libraries for the isolation of rare molecules expressed in the precursors cells or progeny; cells produced by treatment may directly express growth factors or other molecules, and conditioned media is screened in assays for novel activities.

The invention further provides for the isolation, self-renewal and survival of mammalian stem cells and the differentiation of their progeny.

The invention also provides for compositions and methods of preventing or slowing the degeneration of or increasing the numbers of skin cells, in disease states including but not limited to, abnormal proliferation, atrophy, degeneration, toxin-mediated tissue damage, and post-surgical and post-injury tissue regeneration; of preventing or slowing degeneration of or increasing the numbers of cells of the thymus in disorders of the thymus and immune system; and of preventing or slowing the degeneration of neuronal tissue, or increasing the number of neural cells, such as in Parkinson's Disease and Alzheimer's Disease.

The invention also provides for compositions and methods for identifying inhibitors of FGF-23 function, useful in disease states such as cancers and proliferative or differentiation disorders of cells derived from the thymus, neural tissue, skin or placenta.

### Further embodiments of the invention

1. An isolated nucleic acid molecule comprising a polynucleotide selected from the group consisting of:
   (a) a polynucleotide encoding amino acids from about 1 to about 251 of SBQ ID NO:4;
   (b) a polynucleotide encoding amino acids from about 2 to about 251 of SEQ ID NO:4;
   (c) a polynucleotide encoding amino acids from about 1 to about 24 of SEQ ID NO:4;
   (d) a polynucleotide encoding amino acids from about 25 to about 251 of SEQ ID NO:4;
   (e) a polynucleotide encoding amino acids from about 1 to about 175 of SEQ ID NO:4;
   (f) a polynucleotide encoding amino acids from about 1 to about 178 of SEQ ID NO:4;
   (g) a polynucleotide encoding amino acids from about 177 to about 251 of SEQ ID NO:4;
   (h) a polynucleotide encoding amino acids from about 180 to about 251 of SEQ ID NO:4;
   (i) the polynucleotide complement of (a), (b), (c), (d), (e), (f), (g) or (h); and
   (j) a polynucleotide at least 90% identical to the polynucleotide of (a), (b), (c), (d), (e), (f), (g) or (h).
2. An isolated nucleic acid molecule which comprises 20-753 contiguous nucleotides from the coding region of SEQ ID NO:3.
3. The isolated nucleic acid molecule of item 2, which comprises 60-500 contiguous nucleotides from the coding region of SEQ ID NO:3
4. The isolated nucleic acid molecule of item 3, which comprises 200-300 contiguous nucleotides from the coding region of SEQ ID NO:3.
5. An isolated nucleic acid molecule comprising a polynucleotide encoding a polypeptide wherein, except for at least one conservative amino acid substitution, said polypeptide has an amino acid sequence selected from the group consisting of:
   (a) amino acids from about 1 to about 251 of SEQ ID NO:4;
   (b) amino acids from about 2 to about 251 of SEQ ID NO:4;
   (c) amino acids from about 1 to about 24 of SEQ ID NO:4;
   (d) amino acids from about 25 to about 251 of SEQ ID NO:4;
   (e) amino acids from about 1 to about 175 of SEQ ID NO:4;
   (f) amino acids from about 1 to about 177 of SEQ ID NO:4;
   (g) amino acids from about 177 to about 251 of SEQ ID NO:4; and
   (h) amino acids from about 180 to about 251 of SEQ ID NO:4.
6. The isolated nucleic acid molecule of item 1, which is DNA.
7. A method of making a recombinant vector comprising inserting a nucleic acid molecule of item 1 into a vector in operable linkage to a promoter.
8. A recombinant vector produced by the method of item 7.
9. A method of making a recombinant host cell comprising introducing the recombinant vector of item 8 into a host cell.
10. A recombinant host cell produced by the method of item 9.
11. A recombinant method of producing a polypeptide, comprising culturing the recombinant host cell of item 10 under conditions such that said polypeptide is expressed and recovering said polypeptide.
12. An isolated polypeptide comprising amino acids at least 95% identical to amino acids selected from the group consisting of:
   (a) amino acids from about 1 to about 251 of SEQ ID NO:4;
   (b) amino acids from about 2 to about 251 of SEQ ID NO:4;
   (c) amino acids from about 1 to about 24 of SEQ ID NO:4;
   (d) amino acids from about 25 to about 251 of SEQ ID NO:4;
   (e) amino acids from about 1 to about 175 of SEQ ID NO:4;
   (f) amino acids from about 1 to about 177 of SEQ ID NO:4;
   (g) amino acids from about 177 to about 251 of SEQ ID NO:4; and
   (h) amino acids from about 180 to about 251 of SEQ ID NO:4.
13. An isolated polypeptide wherein, except for at least one conservative amino acid substitution, said polypeptide has an amino acid sequence selected from the group consisting of:
   (a) amino acids from about 1 to about 251 of SEQ ID NO:4;
   (b) amino acids from about 2 to about 251 of SEQ ID NO:4;
   (c) amino acids from about 1 to about 24 of SEQ ID NO:4;
   (d) amino acids from about 25 to about 251 of SEQ ID NO:4;
   (e) amino acids from about 1 to about 175 of SEQ ID NO:4;
   (f) amino acids from about 1 to about 177 of SEQ ID NO:4;
   (g) amino acids from about 177 to about 251 of SEQ ID NO:4; and
   (h) amino acids from about 180 to about 251 of SEQ ID NO:4.
14. An isolated polypeptide comprising amino acids selected from the group consisting of:
   (a) amino acids from about 1 to about 251 of SEQ ID NO:4;
   (b) amino acids from about 2 to about 251 of SEQ ID NO:4;
   (c) amino acids from about 1 to about 24 of SEQ ID NO:4;
   (d) amino acids from about 25 to about 251 of SEQ ID NO:4;
   (e) amino acids from about 1 to about 175 of SEQ ID NO:4;
   (f) amino acids from about 1 to about 177 of SEQ ID NO:4;
   (g) amino acids from about 177 to about 251 of SEQ ID NO:4; and
   (h) amino acids from about 180 to about 251 of SEQ ID NO:4.
15. An epitope-bearing portion of the polypeptide of SEQ ID NO:4.
16. The epitope-bearing portion of item 15, which comprises between 10 and 50 contiguous amino acids of SEQ ID NO:4.
17. The epitope-bearing portion of item 5, which comprises amino acids RRHTRSAEDDSERD.
18. The epitope-bearing portion of item 15, which comprises amino acids YHLQIHKNGHVDGAPHQ.
19. An isolated antibody that binds specifically to the polypeptide of item 12.
20. An isolated antibody that binds specifically to the polypeptide of item 13.
21. An isolated antibody that binds specifically to the polypeptide of item 4.
22. A pharmaceutical composition comprising the polypeptide of item 12, in combination with a pharmaceutically acceptable carrier.
23. A method for providing trophic support for cells in a patient in need thereof, the method comprising administering to the patient a composition comprising a polynucleotide encoding the polypeptide of SEQ ID NO:4.
24. The method of item 23 wherein said polynucleotide is administered by implanting cells which express said polynucleotide into the patient, wherein said cells express FGF-23 polypeptide in the patient.
25. The method of item 23 wherein the implanted cells are encapsulated in a semipermeable membrane.
26. The method of item 23 wherein the patient suffers from a condition characterized by dysfunction of or injury to skin cells.
27. The method of item 23 wherein the condition is traumatic injury.
28. The method of item 23 wherein said patient suffers from a condition characterized by inadequate function of placental cells.
29. The method of item 28 wherein said condition is at least one condition selected from the group consisting of congenital defects, fertility, or abnormal growth.
30. The method of item 23 wherein the patient suffers from a condition characterized by inadequate function of the thymus.
31. The method of item 30 wherein said condition is at least one condition selected from the group consisting of leukemia, lymphoma, autoimmune disease, proliferative disorder of the thymus, and differentiation disorder of the thymus.
32. A method for providing trophic support for cells in a patient in need thereof, the method comprising administering to the patient a composition comprising a polypeptide of SEQ ID NO:4.
33. The method of item 28 wherein the patient suffers from a condition characterized by central nervous system disorder.
34. The method of item 29 wherein the condition is selected from the group consisting of Parkinson's disease and Alzheimer's disease.
35. A method of alleviating a disease condition in the brain of a human patient wherein said disease condition is alleviated by at least one method selected from the group consisting of slowing degeneration of, restoring function of, and increasing the number of, functional neuronal cells in said human patient, said method comprising administering to said patient a pharmaceutically effective composition comprising a polypeptide having the amino acid sequence of SEQ ID NO:4.
36. A method of alleviating a disease condition in the thymus of a human patient wherein said disease condition is alleviated by at least one method selected from the group consisting of preventing degeneration of, slowing degeneration of, increasing the number of, functional thymic cells in said human patient, said method comprising administering to said patient a pharmaceutically effective composition comprising a polypeptide having the amino acid sequence of SEQ ID NO:4.
37. A method of alleviating a disease condition in the skin of a human patient wherein said disease condition is alleviated by at least one method selected from the group consisting of preventing degeneration of, slowing degeneration of, and increasing the number of, functional skin cells in said human patient, said method comprising administering to said patient a pharmaceutically effective composition comprising a polypeptide having the amino acid sequence of SEQ ID NO:4.
38. A method of alleviating a disease condition in the placenta of a human patient wherein said disease condition is alleviated by at least one method selected from the group consisting of preventing degeneration of, slowing degeneration of, and increasing the number of, functional placental cells, said method comprising administering to said patient a pharmaceutically effective composition comprising a polypeptide having the amino acid sequence of SEQ ID NO:4.
39. A kit for detecting the presence of mRNA encoding FGF-23 in a sample from a patient, said kit comprising a polynucleotide having at least 20 contiguous nucleotides of the polynucleotide of item 3, packaged in a container.
40. The kit according to item 39 wherein the polynucleotide encodes at least six contiguous amino acids of SEQ ID NO:4.
41. A kit for detecting the presence of FGF-23 polypeptide in a sample from a patient, said kit comprising an antibody according to item 19, packaged in a container.
42. An isolated nucleic acid molecule comprising a polynucleotide selected from the group consisting of:
   (a) a polynucleotide encoding amino acids from about 1 to about 251 of SEQ ID NO:2;
   (b) a polynucleotide encoding amino acids from about 2 to about 251 of SEQ ID NO:2;
   (c) a polynucleotide encoding amino acids from about 1 to about 24 of SEQ ID NO:2;
   (d) a polynucleotide encoding amino acids from about 25 to about 251 of SEQ ID NO:2;
   (e) a polynucleotide encoding amino acids from about 1 to about 175 of SEQ ID NO:2;
   (f) a polynucleotide encoding amino acids from about 1 to about 178 of SEQ ID NO:2;
   (g) a polynucleotide encoding amino acids from about 177 to about 251 of SEQ ID NO:2;
   (h) a polynucleotide encoding amino acids from about 180 to about 251 of SEQ ID NO:2;
   (i) the polynucleotide complement of (a), (b), (c), (d), (e), (f), (g) or (h); and
   (j) a polynucleotide at least 90% identical to the polynucleotide of (a), (b), (c), (d), (e), (f), (g) or (h).
43. An isolated nucleic acid molecule which comprises 20-753 contiguous nucleotides from the coding region of SEQ ID NO:1.
44. The isolated nucleic acid molecule of item 43, which comprises 60-500 contiguous nucleotides from the coding region of SEQ ID NO:1.
45. The isolated nucleic acid molecule of item 44, which comprises 200-300 contiguous nucleotides from the coding region of SEQ ID NO:1.
46. An isolated nucleic acid molecule comprising a polynucleotide encoding a polypeptide wherein, except for at least one conservative amino acid substitution, said polypeptide has an amino acid sequence selected from the group consisting of:
   (a) amino acids from about 1 to about 251 of SEQ ID NO:2;
   (b) amino acids from about 2 to about 251 of SEQ ID NO:2;
   (c) amino acids from about 1 to about 24 of SEQ ID NO:2;
   (d) amino acids from about 25 to about 251 of SEQ ID NO:2;
   (e) amino acids from about 1 to about 175 of SEQ ID NO:2;
   (f) amino acids from about 1 to about 177 of SEQ ID NO:2;
   (g) amino acids from about 177 to about 251 of SEQ ID NO:2; and
   (h) amino acids from about 180 to about 251 of SEQ ID NO:2.
47. The isolated nucleic acid molecule of item 42, which is DNA.
48. A method of making a recombinant vector comprising inserting a nucleic acid molecule of item 42 into a vector in operable linkage to a promoter.
49. A recombinant vector produced by the method of item 48.
50. A method of making a recombinant host cell comprising introducing the recombinant vector of item 49 into a host cell.
51. A recombinant host cell produced by the method of item 50.
52. A recombinant method of producing a polypeptide, comprising culturing the recombinant host cell of item 51 under conditions such that said polypeptide is expressed and recovering said polypeptide.
53. An isolated polypeptide comprising amino acids at least 95% identical to amino acids selected from the group consisting of:
   (a) amino acids from about 1 to about 251 of SEQ ID NO:2;
   (b) amino acids from about 2 to about 251 of SEQ ID NO:2;
   (c) amino acids from about 1 to about 24 of SEQ ID NO:2;
   (d) amino acids from about 25 to about 251 of SEQ ID NO:2;
   (e) amino acids from about 1 to about 175 of SEQ ID NO:2;
   (f) amino acids from about 1 to about 177 of SEQ ID NO:2;
   (g) amino acids from about 177 to about 251 of SEQ ID NO:2; and
   (h) amino acids from about 180 to about 251 of SEQ ID NO:2.
54. An isolated polypeptide wherein, except for at least one conservative amino acid substitution, said polypeptide has an amino acid sequence selected from the group consisting of:
   (a) amino acids from about 1 to about 251 of SEQ ID NO:2;
   (b) amino acids from about 2 to about 251 of SEQ ID NO:2;
   (c) amino acids from about 1 to about 24 of SEQ ID NO:2;
   (d) amino acids from about 25 to about 251 of SEQ ID NO:2;
   (e) amino acids from about 1 to about 175 of SEQ ID NO:2;
   (f) amino acids from about 1 to about 177 of SEQ ID NO:2;
   (g) amino acids from about 177 to about 251 of SEQ ID NO:2; and
   (h) amino acids from about 180 to about 251 of SEQ ID NO:2.
55. An isolated polypeptide comprising amino acids selected from the group consisting of:
   (a) amino acids from about 1 to about 251 of SEQ ID NO:2;
   (b) amino acids from about 2 to about 251 of SEQ ID NO:2;
   (c) amino acids from about 1 to about 24 of SEQ ID NO:2;
   (d) amino acids from about 25 to about 251 of SEQ ID NO:2;
   (e) amino acids from about 1 to about 175 of SEQ ID NO:2;
   (f) amino acids from about 1 to about 177 of SEQ ID NO:2;
   (g) amino acids from about 177 to about 251 of SEQ ID NO:2; and
   (h) amino acids from about 180 to about 251 of SEQ ID NO:2.
56. An epitope-bearing portion of the polypeptide of SEQ ID NO:2.
57. The epitope-bearing portion of item 56, which comprises between 10 and 50 contiguous amino acids of SEQ ID NO:2.
58. An isolated antibody that binds specifically to the polypeptide of item 53.
59. An isolated antibody that binds specifically to the polypeptide of item 54.
60. An isolated antibody that binds specifically to the polypeptide of item 55.

### BRIEF DESCRIPTION OF THE DRAWINGS

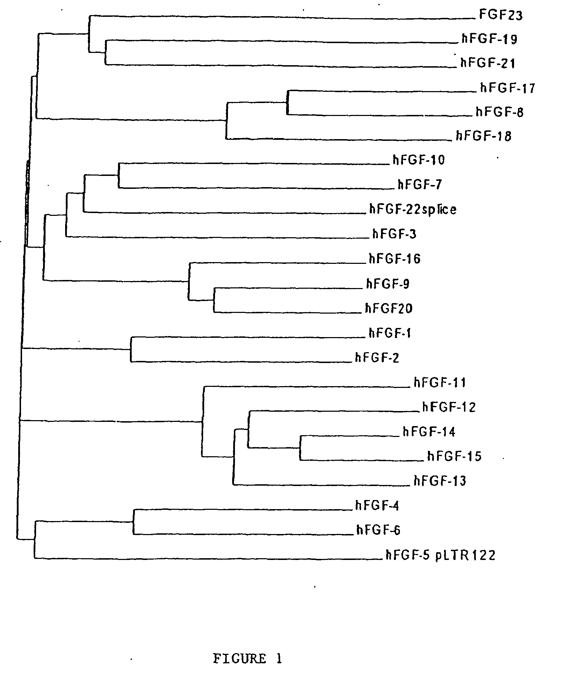

Figure 1. Figure 1 indicates the relationship of FGF-23 to other members of the human FGF gene family.
Figure 2. DNA sequence (SEQ ID NO:1) and amino acid sequence (SEQ ID NO:2) of mouse FGF-23.
Figure 3. DNA sequence (SEQ ID NO:3) and amino acid sequence (SEQ ID NO:4) of human FGF-23.

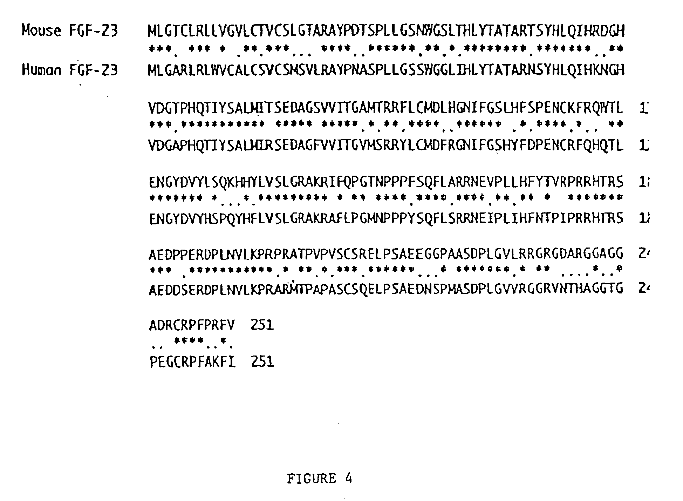

Figure 4. Alignment of the amino acid sequences of human (SEQ ID NO:4) and mouse (SEQ ID NO:2) FGF-23.

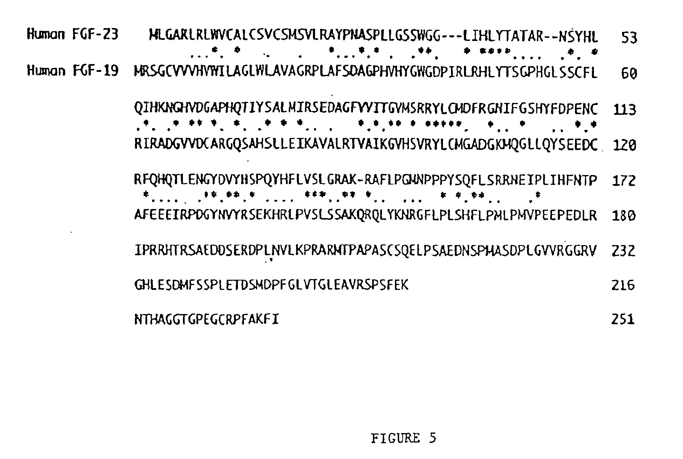

Figure 5. Amino acid sequence comparison of human FGF-23 and human FGF-19. Asterisks indicate identical amino acid residues of the sequences.

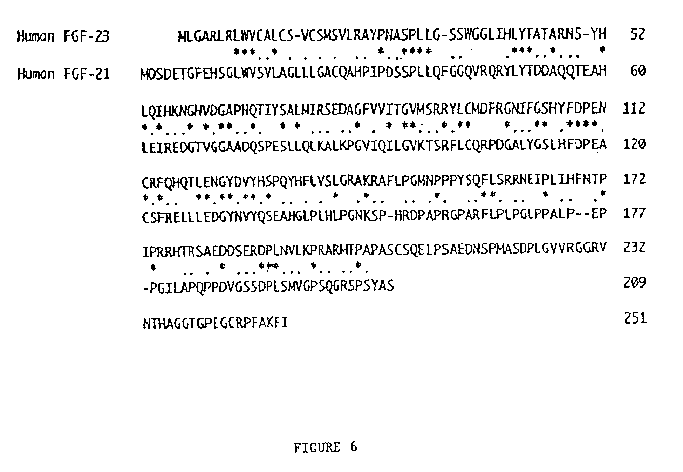

Figure 6. Amino acid sequence comparison of human FGF-23 and human FGF-21.

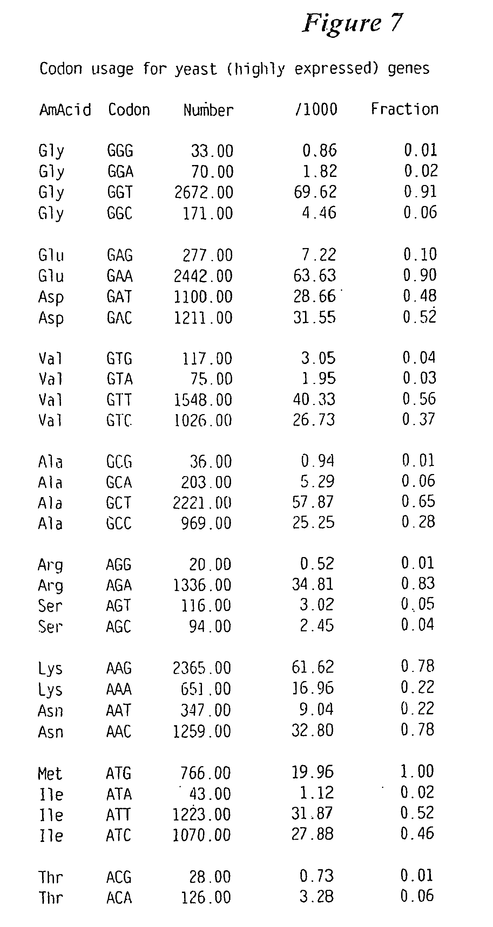

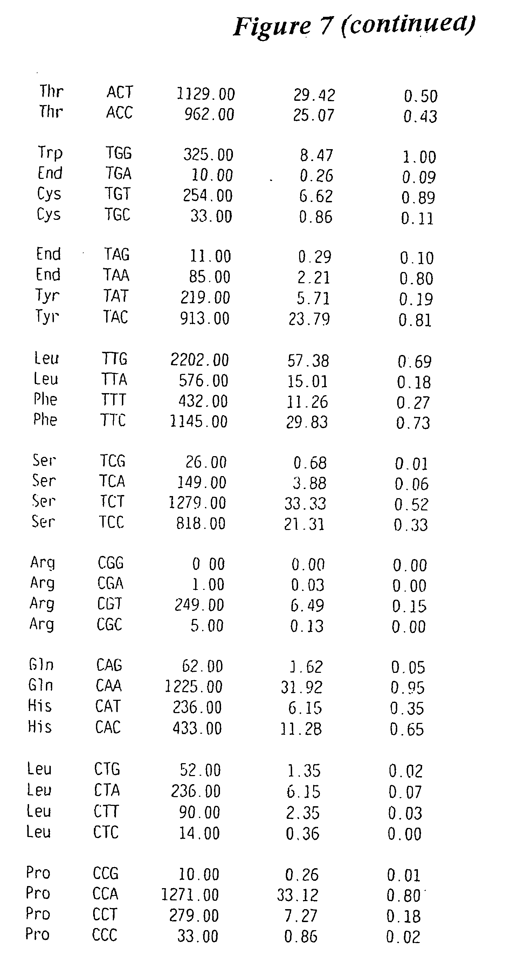

Figure 7. Figure 7 provides codon usage for yeast. The first field of information on each line of the table contains a three-letter code for an amino acid. The second field contains an unambiguous codon for that amino acid. The third field lists the number of occurrences of that codon in the genes from which the table is compiled. The fourth field lists the expected number of occurrences of that codon per 1,000 codons in genes whose codon usage is identical to that compiled in the codon frequency table. The last field contains the fraction of occurrences of the codon in its synonymous codon family.
Figure 8. Figure 8 provides codon usage for Drosophila.
Figure 9. Figure 9 provides codon usage for *E*. *coli.*
Figure 10. Figure 10 provides the chromosomal localization of genes of the human FGF family.
Figure 11. Figure 11 illustrates the detection of recombinant mouse FGF-23 from the culture medium and cell lysate of High Five cells infected with recombinant baculovirus containing the FGF-23 cDNA. The culture medium and cell lysate of the recombinant baculovirus-infected High Five cells were separated by SDS-polyacrylamide gel (12.5%) electrophoresis. Recombinant mouse FGF-23 was detected by Western blotting analysis with anti-E tag antibodies. Prestained Protein Marker Broad Range (New England Biolabs) was used as molecular mass-standard proteins.
Figure 12. Figure 12 illustrates the expression of FGF-23 mRNA in mouse adult tissues. FGF-23 cDNA was amplified from mouse adult tissue cDNAs by real-time quantitative PCR. Copy numbers of FGF-23 were normalized to those of β-actin cDNA in each mouse tissue.
Figure 13. Figure 13 illustrates the localization of FGF-23 mRNA in mouse brain. Consecutive coronal sections of the brain were hybridized with an ³⁵S-labeled mouse FGF-23 antisense (A) or sense probe (B) and exposed to X-ray film. An arrow indicates the ventrolateral thalamic nucleus.
Figure 14. Figure 14 is a schematic diagram of FGF-23 showing the 20 kDa and 7-12 kDa fragments.

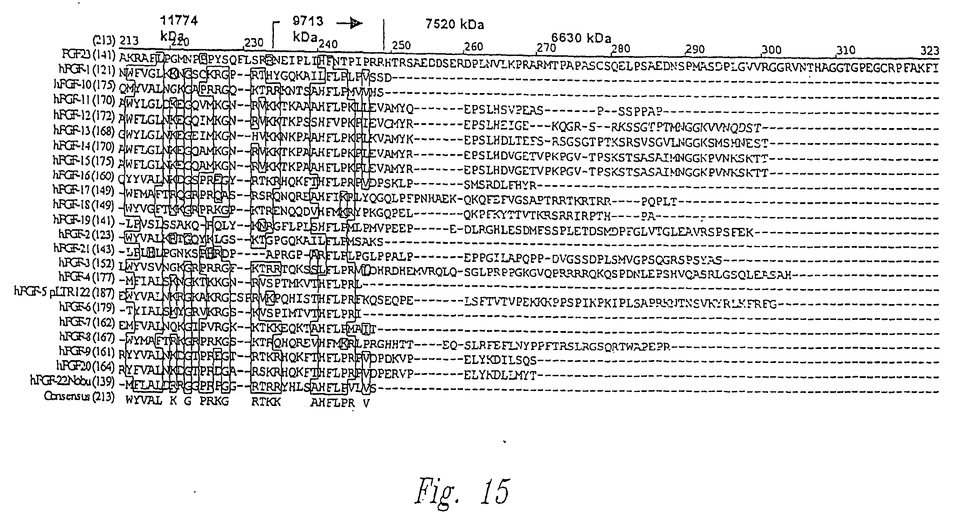

Figure 15. Figure 15 provides an alignment of members of the FGF family, indicating putative cleavage sites for FGF-23.
Figure 16. Figure 16 is a Coomassie-stained gel indicating the molecular weights of FGF-23 cleavage products.

### DETAILED DESCRIPTION OF THE INVENTION

Because of their potent activities for promoting growth, proliferation, survival and differentiation of a wide variety of cells and tissue types, FGFs continue to be pursued as therapeutic agents for a number of different indications, including wound healing, such as musculo-skeletal conditions, for example, bone fractures, ligament and tissue repair, tendonitis, bursitis, etc.; skin conditions, for example, burns, cuts, lacerations, bed sores, slow healing ulcers, etc.; tissue protection, repair, and the induction of angiogenesis during myocardial infarction and ischemia, in the treatment of neurological conditions, for example, neuro-degenerative disease and stroke, in the treatment of eye disease, including macular degeneration, and the like.

The fibroblast growth factor (FGF) proteins identified to date belong to a family of signaling molecules that regulate growth and differentiation of a variety of cell types. The significance of FGF proteins to human physiology and pathology relates in part to their key roles in embryogenesis, in blood vessel development and growth, and in bone growth. *In vitro* experiments have demonstrated a role for FGF in regulating cell growth and division of endothelial cells, vascular smooth muscle cells, fibroblasts, and cardiac and skeletal myocytes. Other members of the FGF family and their biological roles are described in Crossley et al., Development 121:439-451 (1995); Ohuchi et al., Development 124:2235-2244 (1997); Gemel et al., Genomics 35:253-257 (1996); and Ghosh et al., Cell Growth and Differentiation 7:1425-1434 (1996).

FGF proteins are also significant to human health and disease because of a role in cancer cell growth. For example, FGF-8 was identified as an androgen-induced growth factor in breast and prostate cancer cells. (Tanaka et al., FEBS Lett. 363:226-230 (1995) and P.N.A.S 89:8928-8932 (1992)).

New members of the FGF family are described here, wherein the FGF protein is expressed in mammalian skin, thymus, brain and placenta. A polynucleotide encoding the mouse FGF of the invention has the sequence as shown in SEQ ID NO:1. A polynucleotide encoding the human FGF of the invention has the sequence as shown in SEQ ID NO:3. The mouse polynucleotide was identified as encoding a member of the FGF family by the conserved regions throughout the amino acid sequence and by the regions of homology shared by the polynucleotide and genes encoding known FGF proteins.

The inventors believe that FGF-23 is a previously unidentified member of the FGF family. To date, at least 20 human FGF proteins have been identified. In most cases, homologous proteins in other mammals, particularly mice and rats, have also been identified. The human proteins vary to different degrees in terms of amino acid sequence, receptor specificity, tissue expression patterns, and biological activity.

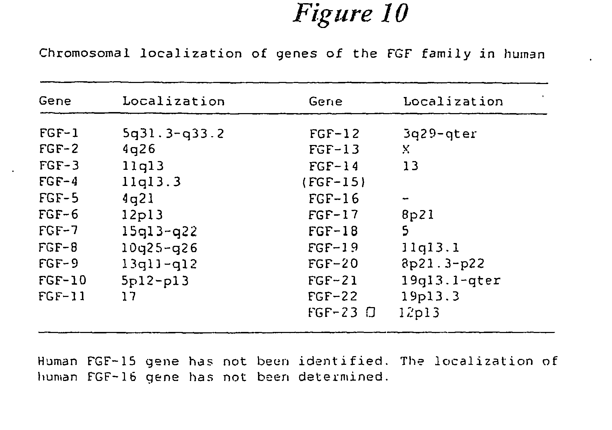

The chromosomal localization of 20 human FGF genes (FGF-1∼FGF-14 and FGF-17∼FGF-22) has been reported (Figure 10). Human FGF genes are localized in various areas of the human genome, except for FGF-3 and FGF-4, which are tandem linked on the chromosome 11. Identification of the chromosomal localization of human FGF genes has often been instrumental in linking human disease and therefore is of particular interest. According to the invention, the human FGF-23 gene is localized on human chromosome 19p13.3 (Figure 10). Interestingly, human FGF-6 gene was also found in the same human genomic DNA. The coding region of the FGF-6 gene is localized at a ∼5.5 upstream site from the coding region of the FGF-23 gene.

The present FGF-23 differs in sequence from all the FGF proteins described to date in publications. As discussed herein, the knowledge about the roles played by various FGF proteins continues to grow, but is by far incomplete.

The present invention adds to this knowledge by disclosing that the FGF of SEQ ID NO:1 is expressed in skin, brain and placenta, and human FGF-23 may play a role in development of and recovery from disease of these tissues. Further, FGF-23 is also expressed in thymus, and therefore may play a role in the development or recovery from disorders of cells derived from the thymus. The invention therefore is based upon the identification, isolation and sequencing of a new fibroblast growth factor, FGF-23.

The present invention further adds to this knowledge by providing products of FGF-23 cleavage, wherein the cleavage occurs at, for example, amino acid 176 or 179. The cleavage can occur during expression, and results in production of a 20 kDa protein and a 7-12 kDa protein, whereas the noncleaved expression product is a 28 kDa protein. Although they are not bound by this mechanism, the inventors believe that the cleavage involves amino acids 176 and 179, both of which are arginine in native human FGF-23.

The cleavage may therefore play a role in disorders of phosphate metabolism, for example as described in Nature Genetics 26:354-348 (2000).

The invention provides compositions and methods for treating disorders of phosphate metabolism, wherein the disorder is a result of cleavage of FGF-23. The composition can comprise an FGF-23, or a polynucleotide encoding FGF-23, wherein the FGF-23 has been mutated to alter or delete the cleavage site. Preferable sites for such mutations include one or both of the arginine residues at positions 176 and 179. Other sites include amino acids near residues 176 or 179, wherein mutation affects cleavage at the nearby residue 176 or 179.

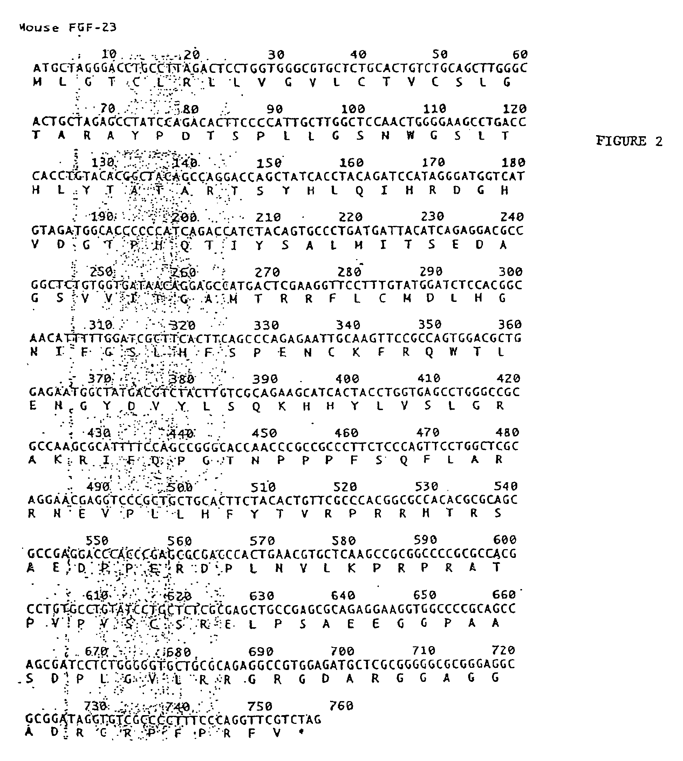

According to the invention, DNA encoding a novel mouse FGF has been identified. The nucleotide sequence of the entire coding region was determined using a genomic fragment from mouse chromosome 6. The nucleotide sequence of the coding region allowed for the elucidation of the complete amino acid sequence of the mouse FGF (251 amino acids) (Figure 2). This protein is tentatively named FGF-23. Two cysteine residues are well conserved in the FGF family, and these amino acids correspond to residues 51 and 113 in mouse FGF-23. A cysteine was found at position 113, and a tyrosine was found at position 51.

A human gene encoding FGF-23 was identified in human placenta. The cDNA comprising the entire coding region of human FGF-23 was amplified by PCR using FGF-specific primers as follows: sense primer: 5' agcaccagccactcagagca 3' (SEQ ID NO:5); antisense primer: 5' cttccagcgaccctagatga 3' (SEQ ID NO:6).

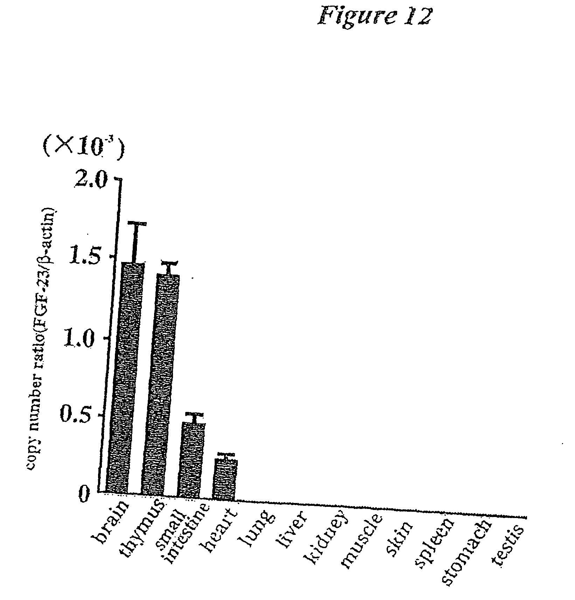

The expression of FGF-23 mRNA in mouse adult tissues was examined by real-time quantitative PCR by a Model 7700 Sequence Detector with forward and reverse primers specific for mouse FGF-23, a TaqMan probe specific for mouse FGF-23, and mouse tissue cDNAs (brain, thymus, small intestine, heart, lung, liver, kidney, muscle, skin, spleen, stomach and testis) as templates. The expression of β-actin mRNA was also examined as a control. The copy numbers of FGF-23 cDNA determined according to the manufacturer's instructions were normalized to those of β-actin cDNA in each tissue (Figure 12). FGF-23 mRNA was found to be mainly expressed in the brain and thymus. However, the expression levels of FGF-23 mRNA are very low in comparison with the expression levels of β-action mRNA. Although mouse FGF-23 cDNA was originally isolated from the skin, the expression of FGF-23 mRNA in the skin was much lower than that of the brain.

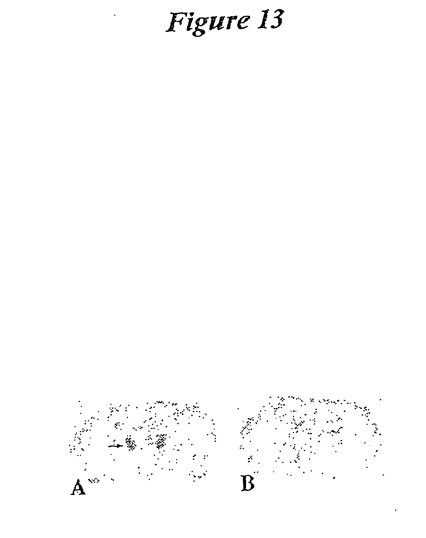

To elucidate roles of FGF-23 in the brain, the expression of FGF-23 mRNA in consecutive coronal section in the brain was examined by *in situ* hybridization with an ³⁵S-labeled antisense or sense mouse FGF-23 cRNA probe. Discrete specific labeling was observed only in the ventrolateral thalamic nucleus (Figure 13). No specific labeling was observed in other brain regions examined. The ventrolateral thalamic nucleus related to the motor system is the major relay for activity from the deep cerebellar nuclei to the motor cortex (Price, J.L., The Rat Nervous System, Academic Press, San Diego, CA, pp. 629-648 (1995)). Ventrolateral thalamotomy interrupts a common circuit involved in the supraspinal component of both physiological and pathological tremors, indicating that the ventrolateral thalamic nucleus is involved in circuits generating physiological tremor. (Duval et al., Exp. Brain Res. 132:216-222 (2000)). FGFs are local signal molecules that act on proximal cells. (Burgess et al., Annu. Rev. Biochem 58:575-606 (1989)). Therefore, FGF-23 is expected to be a unique FGF that plays roles in the ventrolateral thalamic nucleus.

Reference to FGF-23 herein is intended to be construed to include growth factors of any origin which are substantially homologous to and which are biologically equivalent to the FGF-23 characterized and described herein. Such substantially homologous growth factors may be native to any tissue or species and, similarly, biological activity can be characterized in any of a number of biological assay systems.

The term "biologically equivalent" is intended to mean that the compositions of the present invention are capable of demonstrating some or all of the same growth properties in a similar fashion, not necessarily to the same degree as the FGF-23 isolated as described herein or recombinantly produced human FGF-23 of the invention.

By "substantially homologous" it is meant that the degree of homology of human FGF-23 to FGF-23 from any species is greater than that between FGF-23 and any previously reported member of the FGF family.

Sequence identity or percent identity is intended to mean the percentage of same residues between two sequences, referenced to human FGF when determining percent identity with non-human FGF-23, referenced to FGF-23 when determining percent identity with non-FGF-23 growth factors, when the two sequences are aligned using the Clustal method (Higgins et al, Cabios 8:189-191, 1992) of multiple sequence alignment in the Lasergene biocomputing software (DNASTAR, INC, Madison, WI). In this method, multiple alignments are carried out in a progressive manner, in which larger and larger alignment groups are assembled using similarity scores calculated from a series of pairwise alignments. Optimal sequence alignments are obtained by finding the maximum alignment score, which is the average of all scores between the separate residues in the alignment, determined from a residue weight table representing the probability of a given amino acid change occurring in two related proteins over a given evolutionary interval. Penalties for opening and lengthening gaps in the alignment contribute to the score. The default parameters used with this program are as follows: gap penalty for multiple aligriment=10; gap length penalty for multiple alignment=10; k-tuple value in pairwise alignment=1; gap penalty in pairwise alignment=3; window value in pairwise alignment=5; diagonals saved in pairwise alignment=5. The residue weight table used for the alignment program is PAM250 (Dayhoff et al., in Atlas of Protein Sequence and Structure, Dayhoff, Ed., NDRF, Washington, Vol. 5, suppl. 3, p. 345, 1978).

Percent conservation is calculated from the above alignment by adding the percentage of identical residues to the percentage of positions at which the two residues represent a conservative substitution (defined as having a log odds value of greater than or equal to 0.3 in the PAM250 residue weight table). Conservation is referenced to human FGF-23 when determining percent conservation with non-human FGF-23, and referenced to FGF-23 when determining percent conservation with non-FGF-23 growth factors. Conservative amino acid changes satisfying this requirement are: R-K; E-D, Y-F, L-M; V-I,Q-H.

The invention provides FGF-23 proteins or variants thereof having one or more polymers covalently attached to one or more reactive amino acid side chains. By way of example, not limitation, such polymers include polyethylene glycol (PEG), which can be attached to one or more free cysteine sulfhydryl residues, thereby blocking the formation of disulfide bonds and aggregation when the protein is exposed to oxidizing conditions. In addition, pegylation of FGF-23 proteins and/or muteins is expected to provide such improved properties as increased half-life, solubility, and protease resistance. FGF-23 proteins and/or muteins may alternatively be modified by the covalent addition of polymers to free amino groups such as the lysine epsilon or the N-terminal amino group. Preferred cysteines and lysines for covalent modification will be those not involved in receptor or heparin binding or in proper protein folding. It will be apparent to one skilled in the art that the methods for assaying FGF-23 biochemical and/or biological activity may be employed in order to determine if modification of a particular amino acid residue affects the activity of the protein as desired.

It may be advantageous to improve the stability of FGF-23 by modifying one or more protease cleavage sites. Thus, the present invention provides FGF-23 variants in which one or more protease cleavage site has been altered by, for example, substitution of one or more amino acids at the cleavage site in order to create an FGF-23 variant with improved stability. Such improved protein stability may be beneficial during protein production and/or therapeutic use.

Suitable protease cleavage sites for modification are well known in the art and likely will vary depending on the particular application contemplated. For example, typical substitutions would include replacement of lysines or arginines with other amino acids such as alanine. The loss of activity, such as receptor binding or heparin binding, can be tested for as described herein.

FGF-23 can also include hybrid and modified forms of FGF-23 including fusion proteins and FGF-23 fragments and hybrid and modified forms in which certain amino acids have been deleted or replaced and modifications such as where one or more amino acids have been changed to a modified amino acid or unusual amino acid and modifications such as glycosylations so long as the hybrid or modified form retains the biological activity of FGF-23. Fusion proteins can consist of the FGF-23 of the invention or fragment thereof and a signal sequence of a heterologous protein to promote secretion of the protein product.

Fusion proteins comprising FGF-23 or a biologically active or antigenic fragment thereof can be produced using methods known in the art. Such fusion proteins can be used therapeutically or can be produced in order to simplify the isolation and purification procedures. Histidine residues can be incorporated to allow immobilized metal affinity chromatography purification. Residues EQKLISEEDL contain the antigenic determinant recognized by the myc monoclonal antibody and can be incorporated to allow myc monoclonal antibody-based affinity purification. A thrombin cleavage site can be incorporated to allow cleavage of the molecule at a chosen site; a preferred thrombin cleavage site consists of residues LVPRG. Purification of the molecule can be facilitated by incorporating a sequence, such as residues SAWRHPQFGG, which binds to paramagnetic streptavidin beads. Such embodiments are described in WO 97/25345, which is incorporated by reference.

The invention also includes fragments of FGF-23. Preferred fragments of SEQ ID NO:4 and 2 include: amino acids from about 1 to about 251; about 2 to about 251; about 25 to about 251; and about 1 to about 24. Such fragments can be prepared from the proteins by standard biochemical methods, or by expressing a polynucleotide encoding the fragment.

FGF-23 has a typical signal sequence of 24 amino acids at the N-terminus, suggesting that it is a secreted molecule. Recombinant mouse FGF-23 was efficiently secreted by High Five insect cells infected with recombinant baculovirus containing the cDNA, confirming that FGF-23 is a secreted protein.

Also included with the scope of the invention are FGF-23 molecules that differ from native FGF-23 by virtue of changes in biologically active sites.

Also included within the meaning of substantially homologous is any FGF-23 which may be isolated by virtue of cross-reactivity with antibodies to the FGF-23 described herein or whose encoding nucleotide sequences including genomic DNA, mRNA or cDNA may be isolated through hybridization with the complementary sequence of genomic or subgenomic nucleotide sequences or cDNA of the FGF-23 herein or fragments thereof. It will also be appreciated by one skilled in the art that degenerate DNA sequences can encode human FGF-23 and these are also intended to be included within the present invention, as are mammalian allelic variants of FGF-23.

Growth factors are thought to act at specific receptors. According to the invention, FGF-23 and as yet unknown members of this family of growth factors act through specific receptors having distinct distributions as has been shown for other growth factor families.

A preferred hFGF-23 of the present invention has been identified. Also preferred is hFGF-23 prepared by recombinant DNA technology. By "pure form" or "purified form" or "substantially purified form" it is meant that an FGF-23 composition is substantially free of other proteins which are not FGF-23.

Included within the scope of the invention are polynucleotides, including DNA and RNA, with 80% homology to SEQ ID NO:1 or SEQ ID NO:3; preferably at least 85% homology, more preferably at least 90% homology, most preferably 95% homology. Polynucleotides with 96%, 97%, 98%, and 99% homology to SEQ ID NO:1 or 3 are also included. Percent homology is calculated using methods known in the art. A nonlimiting example of such a method is the Smith-Waterman homology search algorithm as implemented in MPSRCH program (Oxford Molecular), using an affine gap search with a gap open penalty of 12 and a gap extension penalty of 1.

FGF-23 can also include hybrid and modified forms of FGF-23 including fusion proteins and FGF-23 fragments and hybrid and modified forms in which certain amino acids have been deleted or replaced and modifications such as where one or more amino acids have been changed to a modified amino acid or unusual amino acid and modifications such as glycosylations so long as the hybrid or modified form retains the biological activity of FGF-23. By retaining the biological activity, it is meant that the ability of FGF-23 to promote the growth, survival or differentiation of responsive cells is preserved, although not necessarily at the same level of potency as that of the FGF-23 isolated as described herein or that of the recombinantly produced FGF-23.

The invention also relates to cleavage products of FGF-23, including a ∼20 kDa form and a ∼7-12 kDa form. Such products are obtained by cleavage of FGF-23 at position 176 or 179. Noncleavable forms are also provided, wherein the arginine at position 176 and/or at position 179 is mutated, deleted, or replaced, or one or more amino acids near these sites is mutated, deleted, or replaced, thereby affecting cleavage at one or both of these sites.

Recombinant human FGF-23 may be made by expressing the DNA sequences encoding FGF-23 in a suitable transformed host cell. Using methods well known in the art, the DNA encoding FGF-23 may be linked to an expression vector, transformed into a host cell and conditions established that are suitable for expression of FGF-23 by the transformed cell.

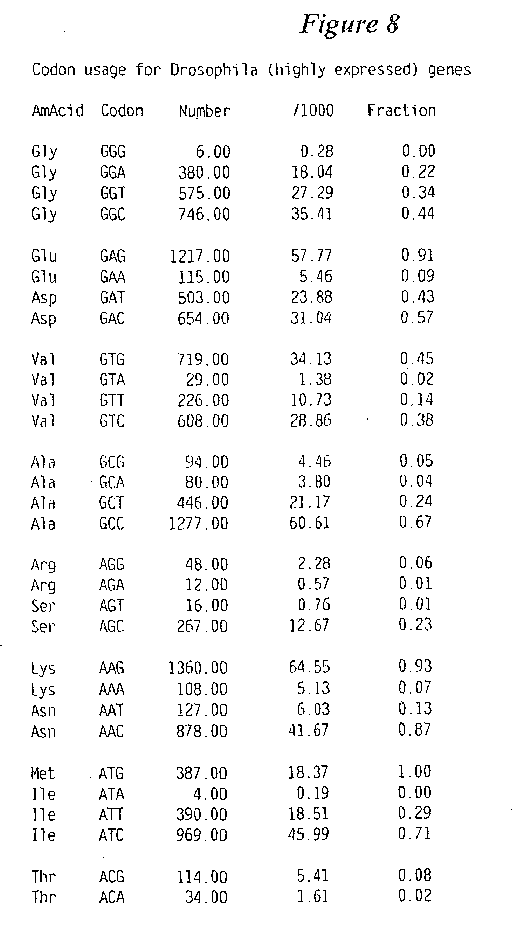

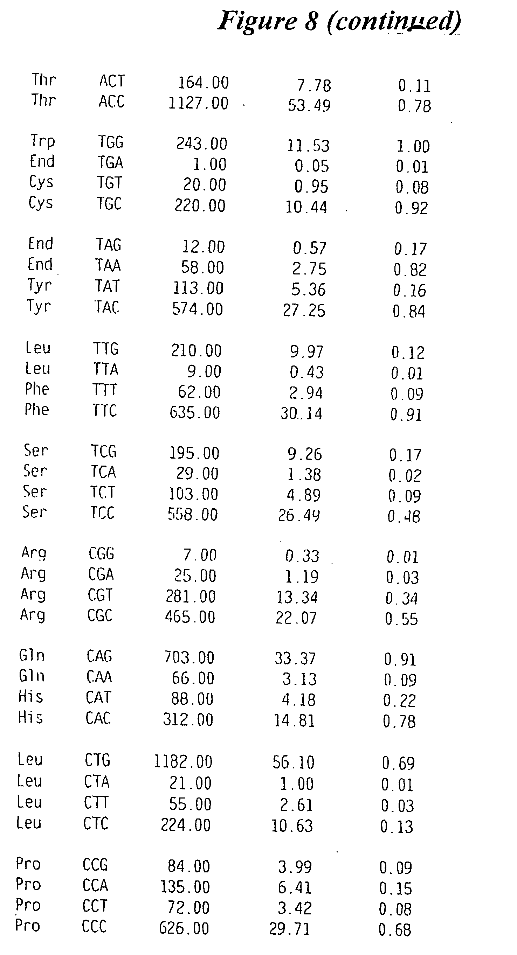

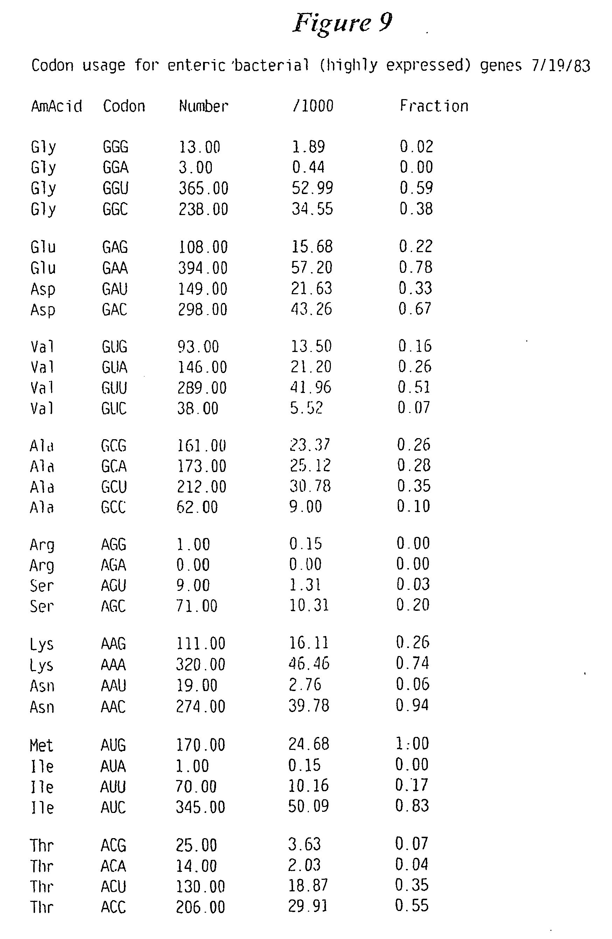

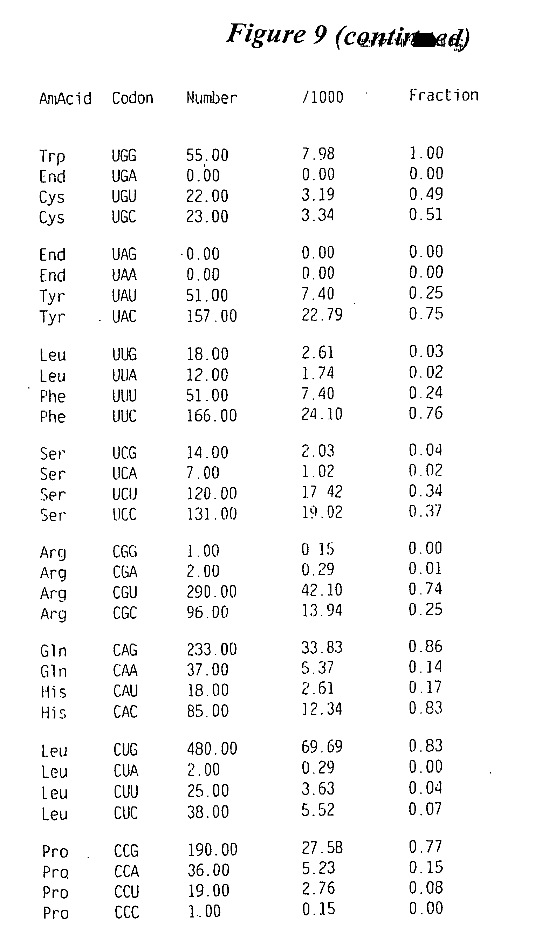

The DNA encoding FGF-23 can be engineered to take advantage of preferred codon usage of host cells. Codon usage in *Pseudomonas aeruginosa* is described in, for example, West et al., Nucleic Acids Res. 11:9323-9335 (1988). Codon usage in *Saccharomyces cerevisiae* is described in, for example, Lloyd et al., Nucleic Acids Res. 20:5289-5295 (1992). Codon preference in Corynebacteria and a comparison with *E*. *coli* preference is provided in Malubres et al., Gene 134:15-24 (1993). Codon usage in *Drosophila melanogaster* is described in, for example, Akashi, Genetics 136:927-935 (1994). Codon usage in yeast is also shown in Figure 7, codon usage in Drosophila is shown in Figure 8, and codon usage for *E*. *coli* is shown in Figure 9.

Any suitable expression vector may be employed to produce recombinant human FGF-23 such as expression vectors for use in insect cells. Baculovirus expression systems can also be employed. A preferable method is expression in insect cells, such as Tr5 or Sf9 cells, using baculovirus vector.

The present invention includes nucleic acid sequences including sequences that encode human FGF-23. Also included within the scope of this invention are sequences that are substantially the same as the nucleic acid sequences encoding FGF-23. Such substantially the same sequences may, for example, be substituted with codons more readily expressed in a given host cell such as *E*. *coli* according to well known and standard procedures. Such modified nucleic acid sequences are included within the scope of this invention.

Specific nucleic acid sequences can be modified by those skilled in the art and, thus, all nucleic acid sequences that code for the amino acid sequences of FGF-23 can likewise be so modified. The present invention thus also includes nucleic acid sequence which will hybridize with all such nucleic acid sequences, or complements of the nucleic acid sequences where appropriate, and encode a polypeptide having the cell survival, growth or differentiation activity of FGF-23. The present invention also includes nucleic acid sequences that encode polypeptides that have cell survival promoting activity and that are recognized by antibodies that bind to FGF-23. Preferred methods and epitopes for raising antibodies are described in Example 7.

The present invention also encompasses vectors comprising expression regulatory elements operably linked to any of the nucleic acid sequences included within the scope of the invention. This invention also includes host cells of any variety that have been transformed with vectors comprising expression regulatory elements operably linked to any of the nucleic acid sequences included within the scope of the present invention.

Methods are also provided herein for producing FGF-23. Preparation can be by isolation from conditioned medium from a variety of cell types so long as the cell type produces FGF-23. A second and preferred method involves utilization of recombinant methods by isolating or obtaining a nucleic acid sequence encoding FGF-23, cloning the sequence along with appropriate regulatory sequences into suitable vectors and cell types, and expressing the sequence to produce FGF-23.

Although FGF-23 has been described on the basis of its expression level in skin, thymus, brain, and placenta, this factor may act on other cell types as well. It is likely that FGF-23 will act on cells to promote their survival, growth, differentiation state or function. This expectation is based upon the activity of known growth factors. Members of the FGF family act on many cell types of different function and embryologic origin, even when their expression is limited to one or a few tissues.

The inventors herein have identified that FGF-23 is expressed in skin. This suggests a role for FGF-23 in, for example, precancerous lesions, repair following inflammatory disease, trauma, or toxin-related injury, and other diseases of the skin. Further, FGF-23 is also expressed in thymus. This suggests a role for FGF-23 in, for example, disorders of cells such as immune cells derived from the thymus, for example, autoimmune disorders, leukemias and lymphomas, immune deficiency states, and the like.

Several FGFs are expressed in brain and expected to play important roles as neutrophic factors. FGF-1 and FGF-2 are abundant in brain (Gospodarowicz, D., Methods Enzymol. 147:106-119 (1987)) and exert survival enhancing effects on primary cultures from various regions of the brain (Walicke, P.A., J. Neurosci. 8:2618-2627 (1988)). FGF-1 is expressed predominantly in motor and sensory neurons of the midbrain and brainstem (Elde, R. et al., Neuron 7:349-364 (1991)). In contrast, FGF-2 is preferentially expressed in neurons in restricted regions including the cingulate cortex, industium grieum, fasciola cinererum and hippocampus, and in astrocytes in widespread regions of the brain (Emoto, N. et al., Growth Factors 2:21-29 (1989), Woodward, W.R et al., J. Neurosci. 12:142-152 (1992)). FGF-5 is weakly expressed in the cerebral cortex, hippocampus and thalamus (Haub, O. et al., Proc. Natl. Acad Sci. USA 87:8022-8026 (1990)). FGF-9 and FGF-11 to FGF-14 are expressed in neurons of restricted regions including the hippocampus, thalamus, midbrain and brainstem (Yamamoto, S. et al., Biochim. Biophys. Acta 1398:38-41 (1998)).

Degeneration of dopaminergic neurons in the substantia nigra causes Parkinson's disease (Fallon, J.H., and Loughlin, S.E., The Rat Nervous System, 2nd Ed., Academic Press, San Diego, CA, pp. 215-238 (1995)). Therefore, neurotrophic factors for dopaminergic neurons in the substantia nigra have received substantial attention. GDNF, Persephin, Artemin, BDNF, and NT-3 enhance survival of midbrain dopaminergic neurons (Lin, L.-F.H. et al., Science 260:1130-1132 (1993), Milbrandt, J. et al., Neuron 20:245-253 (1998), Baloh, R.H. et al., Neuron 21:1291-1302 (1998), Hyman, C. et al., Nature 350:230-232 (1991), Hyman, C. et al., J. Neurosci. 14:335-347 (1994)). However, their expression is not restricted to the substantia nigra (Pochon, N.A. et al., Eur. J. Neurosci. 9:463-471 (1997), Milbrandt, J. et al., Neuron 20:245-253 (1998), Baloh, R.H. et al., Neuron 21:1291-1302 (1998), Ernfors, P. et al., Neuron 5:511-526 (1990)). It is an important finding of the invention that FGF-23 is expressed in the brain.

It is believed that dopamine neurons are dysfunctional for, perhaps, years, before they are irreversibly damaged. Thus, agents such as FGF-23 may be useful in preventing cell death or restoring function (Dunnett, S.B. et al., Nature 399:A32-A39 (1999)). The FGF-23 may be administered using gene transfer methods to block degeneration. Such methods have been used with neurotrophic factor GDNF (glial cell line-derived neutrophic factor). In a rat Parkinson's model, nanogram amounts of BDNF and GDNF were measured from transduced cells, and the neuroprotective effect was in the order of 40-70% rescue of nigral dopamine neurons. Thus, transplants using fibroblasts or fibroblast cell lines engineered to secrete FGF-23 of the invention can allow secretion of the factor and rescue of nigral dopamine neurons. Alternatively, injection of the striatum or the substantia nigra region with viral vectors carrying the FGF-23 gene may also have a neuroprotective effect.

In Parkinson's Disease, neuronal degeneration in the substantial nigra generally is slow and protracted. This suggests that early intervention could block or slow down the degenerative process, perhaps up to 4 or 5 years before clinical symptoms appear. A decline in striatal dopamine function can be detected by PET and SPECT imaging before the appearance of clinical symptoms, providing an opportunity for neuroprotective intervention at this early stage.

The FGF-23 of the invention may also find use in treating other neurodegenerative diseases such as Alzheimer's disease. Additional conditions amenable to treatment include stroke, brain trauma due to physical, chemical or environmental stimuli, toxic insults to the central nervous system, and any other CNS or neurological disorder in which blocking, slowing or reversing of the degenerative process would alleviate the disease or condition.

Other diseases which may be treated according to the invention are Crohn's disease, healing of intestinal wounds, ulcers, inflammation, injuries and surgical anastomoses, motility and absorption disorders, and congenital malformations of the intestine.

The nucleotide and amino acid sequences of FGF-23 have been determined, according to the invention. The invention also provides naturally occurring cleavage products of FGF-23, as described in Example 2. The ability of FGF-23 to be cleaved naturally indicates that the cleavage products may play a role in biological processes in the cell. One such process relates to phosphate metabolism. The results disclosed herein regarding FGF-23 cleavage products are consistent with a recent report that autosomal dominant hypophosphatemic rickets (ADHR) is associated with mutations in FGF-23, specifically at arginine 176 (R176Q) and arginine 179 (R179W) (The ADHR Consortium, Nature Genetics 26:345-348 (2000)). Although they are not bound by the mechanism, the inventors believe that such mutations occur at the cleavage site disclosed herein, and that FGF-23 having a mutation at R176 or R179 would be incapable of cleavage.

According to the invention, FGF-23, when expressed in baculovirus, is cleaved into two pieces (processed). Based on the apparent sizes of the cleavage products on a gel, the predicted cleavage site is believed to be near residues 176 and 179. These arginines, particularly R176, are consistent with being protease sites. The invention therefore provides fragments of FGF-23, including but not limited to amino acids from about 1 to about 175 of SEQ ID NO:4; amino acids from about 1 to about 178 of SEQ ID NO:4; amino acids from about 177 to about 251 of SEQ ID NO:4; and amino acids from about 180 to about 251 of SEQ ID NO:4, as well as polynucleotides encoding these fragments.

Further, the region of FGF-23 which is homologous to other members of the FGF family ends exactly at these residues, with the remainder of the FGF-23 gene being a C-terminal extension without homology to other FGFs or to other proteins by BLAST searching. Thus, the predicted cleavage site would generate an FGF-homologous fragment, and the C terminus. Mutations may play a role in bone disease by preventing proteolytic processing of FGF-23 at these, or nearby, sites. Proteolytic cleavage may be required for activity, and mutation of these sites may cause a loss-of-function phenotype, leading to bone disease.

In addition to ADHR, there is a similar human bone disease, XLH (X-linked Hypophosphatemic rickets) which has been mapped to an endopeptidase, called PHEX. The substrate of PHEX may be FGF-23, at the cleavage site that yields the FGF-23 fragments disclosed herein.

The invention therefore provides variants of FGF-23 with mutations at these sites to render FGF-23 uncleavable, and the variants are either inactive and could be used as dominant negative inhibitors of FGF-23, or are hyperactive and could be used for treating disorders responsive to FGF-23. The inability of mutated FGF-23 to undergo cleavage may contribute to the disrupted phosphate metabolism of rickets and as seen in other disorders. Alternatively, cleavage of these sites may be necessary to inactivate FGF-23, and mutation at these sites may cause gain-of function phenotype leading to phosphate wasting and bone disease.

The present invention also includes therapeutic or pharmaceutical compositions comprising FGF-23 in an effective amount for treating patients with thymic disease including immune disorders characterized by thymic dysfunction, and a method comprising administering a therapeutically effective amount of FGF-23. These compositions and methods are useful for treating a number of diseases. The compositions and methods herein can also be useful to prevent degeneration and/or promote survival in other tissues as well, such as promoting angiogenesis, neuronal survival, wound healing, and the like. One skilled in the art can readily use a variety of assays known in the art to determine whether FGF-23 would be useful in promoting survival or functioning in a particular cell type.

Because FGF-23 is expressed in skin, it may be useful in wound healing, and in diseases of the skin involving abnormal proliferation, atrophy, degeneration, or as a result of toxic insult. FGF-23 may also be useful in conditions related to placental function, such as fertility, congenital defects, and disorders of the placenta.

In certain circumstances, it may be desirable to modulate or decrease the amount of FGF-23 expressed. Thus, in another aspect of the present invention, FGF-23 anti-sense oligonucleotides can be made and a method utilized for diminishing the level of expression of FGF-23 by a cell comprising administering one or more FGF-23 antisense oligonucleotides. By FGF-23 anti-sense oligonucleotides reference is made to oligonucleotides that have a nucleotide sequence that interacts through base pairing with a specific complementary nucleic acid sequence involved in the expression of FGF-23 such that the expression of FGF-23 is reduced. Preferably, the specific nucleic acid sequence involved in the expression of FGF-23 is a genomic DNA molecule or mRNA molecule that encodes FGF-23. This genomic DNA molecule can comprise regulatory regions of the FGF-23 gene, or the coding sequence for mature FGF-23 protein. The term complementary to a nucleotide sequence in the context of FGF-23 antisense oligonucleotides and methods therefor means sufficiently complementary to such a sequence as to allow hybridization to that sequence in a cell, i.e., under physiological conditions. The FGF-23 antisense oligonucleotides preferably comprise a sequence containing from about 8 to about 100 nucleotides and more preferably the FGF-23 antisense oligonucleotides comprise from about 15 to about 30 nucleotides. The FGF-23 antisense oligonucleotides can also contain a variety of modifications that confer resistance to nucleolytic degradation such as, for example, modified internucleoside linages (Uhlmann and Peyman, Chemical Reviews 90:543-548 1990; Schneider and Banner, Tetrahedron Lett. 31:335, 1990 which are incorporated by reference), modified nucleic acid bases and/or sugars and the like.

The therapeutic or pharmaceutical compositions of the present invention can be administered by any suitable route known in the art including for example topical, intravenous, subcutaneous, intramuscular, transdermal, intrathecal or intracerebral. Administration can be either rapid as by injection or over a period of time as by slow infusion or administration of slow release formulation.

FGF-23 can also be linked or conjugated with agents that provide desirable pharmaceutical or pharmacodynamic properties. For example, FGF-23 can be coupled to any substance known in the art to promote penetration or transport across the blood-brain barrier such as an antibody to the transferring receptor, and administered by intravenous injection (*see*, for example, Friden et al., Science 259:373-377,1993 which is incorporated by reference). Furthermore, FGF-23 can be stably linked to a polymer such as polyethylene glycol to obtain desirable properties of solubility, stability, half-life and other pharmaceutically advantageous properties. (*See*, for example, Davis et al., Enzyme Eng. 4:169-73, 1978; Burnham, Am. J. Hosp. Pharm. 51:210-218, 1994 which are incorporated by reference.)

The compositions are usually employed in the form of pharmaceutical preparations. Such preparations are made in a manner well known in the pharmaceutical art. One preferred preparation utilizes a vehicle of physiological saline solution, but it is contemplated that other pharmaceutically acceptable carriers such as physiological concentrations of other non-toxic salts, five percent aqueous glucose solution, sterile water or the like may also be used. It may also be desirable that a suitable buffer be present in the composition. Such solutions can, if desired, be lyophilized and stored in a sterile ampoule ready for reconstitution by the addition of sterile water for ready injection. The primary solvent can be aqueous or alternatively non-aqueous. FGF-23 can also be incorporated into a solid or semi-solid biologically compatible matrix which can be implanted into tissues requiring treatment.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Such excipients are those substances usually and customarily employed to formulate dosages for parenteral administration in either unit dosage or multi-dose form or for direct infusion into the cerebrospinal fluid by continuous or periodic infusion.

Dose administration can be repeated depending upon the pharmacokinetic parameters of the dosage formulation and the route of administration used.

It is also contemplated that certain formulations containing FGF-23 are to be administered orally. Such formulations are preferably encapsulated and formulated with suitable carriers in solid dosage forms. Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, gelatin, syrup, methyl cellulose, methyl- and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic degradation and promote absorption such as, for example, surface active agents.

Depending on the treatment regimen contemplated, it may be desired to control the rate of release of FGF-23 protein or variant thereof to provide long-term treatment while minimizing the frequency of administration. Such treatment regimens may be desired, for example, where the FGF-23 protein is found to be relatively unstable such that the localized concentration of active protein is at an efficacious level for an insufficient period of time. Thus, for example, for certain diseases, it may not be desired or practical to perform repeated and frequent injections. The major advantages of such sustained release systems include targeted local delivery of drugs at a constant rate, less drug required to treat the disease state, minimization of possible side effects, and enhanced efficacy of treatment. Also, these forms of delivery systems are capable of protecting drugs that are unstable *in vivo* and that would normally require a frequent dosing interval. Under such circumstances, sustained release may be achieved by one of the methods readily available in the art such as the encapsulation of FGF-23 conjugated heparin-Sepharose beads to form heparin-alginate microspheres or the preparation of FGF-23 PLG microspheres.

Heparin-alginate microspheres have been successfully employed for the delivery of Basic Fibroblast Growth Factor to tissue (Lopez et al., Journal of Pharmacology and Experimental Therapeutics 282(1):385-390 (1997)). Similarly, Alginate/heparin-Sepharose microspheres and films have been used as drug carriers to control the release of a basic FGF-saponin conjugate in order to control its release in small doses. Addition of heparin to solutions of bFGF prevents losses in activity that accompany changes in pH or elevation in temperature. *See*, for example, Gospodarowicz et al., J. Cell. Physiol. 128:475-484 (1986).

Binding of FGF-23 to heparin may be employed in order to enhance its stability either during *in vivo* expression or administration or *in vitro* during various stages of protein purification. Thus, by the present invention, heparin may be added to a solution of FGF-23 and the activity assayed by the methods disclosed herein.

FGF-23 bound heparin-Sepharose beads may be encapsulated into calcium alginate microspheres to permit the controlled release of the heparin-stabilized FGF-23 protein. For example, microspheres may be constructed by dropping a mixed solution of sodium alginate with FGF-23 bound heparin-Sepharose beads into a hardening solution of calcium chloride. Spheres are formed instantaneously as the mixture enters the hardening solution. The size of the microsphere may be adjusted by passing the FGF-23 bound heparin-Sepharose beads through a cylinder of reduced cross-sectional area such as through a hypodermic needle.

Encapsulation efficiency may be determined by comparing the amount of encapsulated growth factor with that initially present in solution. For example, the FGF-23 may be stripped from the heparin-Sepharose beads with a solution of 3 M NaCl and functional activity assays may be performed.

The specific dose is calculated according to the approximate body weight or body surface area of the patient or the volume of body space to be occupied. The dose will also be calculated dependent upon the particular route of administration selected. Further refinement of the calculations necessary to determine the appropriate dosage for treatment is routinely made by those of ordinary skill in the art. Such calculations can be made without undue experimentation by one skilled in the art in light of the activity disclosed herein in assay preparations of target cells. Exact dosages are determined in conjunction with standard dose-response studies. It will be understood that the amount of the composition actually administered will be determined by a practitioner, in the light of the relevant circumstances including the condition or conditions to be treated, the choice of composition to be administered, the age, weight, and response of the individual patient, the severity of the patient's symptoms, and the chosen route of administration.

In one embodiment of this invention, FGF-23 may be therapeutically administered by implanting into patients vectors or cells capable of producing a biologically-active form of FGF-23 or a precursor of FGF-23, *i.e.,* a molecule that can be readily converted to a biological-active form of FGF-23 by the body. In one approach cells that secrete FGF-23 may be encapsulated into semipermeable membranes for implantation into a patient. The cells can be cells that normally express FGF-23 or a precursor thereof or the cells can be transformed to express FGF-23 or a precursor thereof. It is preferred that the cell be of human origin and that the FGF-23 be human FGF-23 when the patient is human. However, the formulations and methods herein can be used for veterinary as well as human applications and the term "patient" as used herein is intended to include human and veterinary patients.

Cells can be grown *ex vivo* for use in transplantation or engraftment into patients (Muench et al., Leuk. & Lymph. 16:1-11, 1994 which is incorporated by reference). In another embodiment of the present invention, FGF-23 is used to promote the *ex vivo* expansion of a cells for transplantation or engraftment. Current methods have used bioreactor culture systems containing factors such as erythropoietin, colony stimulating factors, stem cell factor, and interleukins to expand hematopoietic progenitor cells for erythrocytes, monocytes, neutrophils, and lymphocytes (Verfaillie, Stem Cells 12:466-476, 1994 which is incorporated by reference). These stem cells can be isolated from the marrow of human donors, from human peripheral blood, or from umbilical cord blood cells. The expanded blood cells are used to treat patients who lack these cells as a result of specific disease conditions or as a result of high dose chemotherapy for treatment of malignancy (George, Stem Cells 12(Suppl 1):249-255, 1994 which is incorporated by reference). In the case of cell transplant after chemotherapy, autologous transplants can be performed by removing bone marrow cells before chemotherapy, expanding the cells *ex vivo* using methods that also function to purge malignant cells, and transplanting the expanded cells back into the patient following chemotherapy (for review, *see* Rummel and Van Zant, J. Hematotherapy 3:213-218, 1994 which is incorporated by reference).

In a number of circumstances it would be desirable to determine the levels of FGF-23 in a patient. The identification of FGF-23 along with the present report showing expression of FGF-23 provides the basis for the conclusion that the presence of FGF-23 serves a normal physiological function related to cell growth and survival. Endogenously produced FGF-23 may also play a role in certain disease conditions.

Given that FGF-23 is expressed in skin, brain, thymus and placenta, it is likely that the level of FGF-23 may be altered in a variety of conditions and that quantification of FGF-23 levels would provide clinically useful information. Furthermore, in the treatment of degenerative conditions, altered physiological function or in recovery from injury to the skin, brain, or thymic cells, or during pregnancy if the condition of the placenta is involved, compositions containing FGF-23 can be administered and it would likely be desirable to achieve certain target levels of FGF-23 in sera or in any desired tissue compartment. It would, therefore, be advantageous to be able to monitor the levels of FGF-23 in a patient. Accordingly, the present invention also provides methods for detecting the presence of FGF-23 in a sample from a patient.

The term "detection" as used herein in the context of detecting the presence of FGF-23 in a patient is intended to include determining the amount of FGF-23 or the ability to express an amount of FGF-23 in a patient, distinguishing FGF-23 from other growth factors, the estimation of prognosis in terms of probable outcome of a degenerative disease and prospect for recovery, monitoring the FGF-23 levels over a period of time as a measure of status of the condition, and monitoring FGF-23 levels for determining a preferred therapeutic regimen for the patient.

To detect the presence of FGF-23 in a patient, a sample is obtained from the patient. The sample can be a tissue biopsy sample or a sample of blood, plasma, serum, CSF or the like. When assessing the levels of FGF-23 in the skin, brain, thymus or placenta, a preferred sample is a sample taken from these tissues or from veins draining these tissues.

In some instances it is desirable to determine whether the FGF-23 gene is intact in the patient or in a tissue or cell line within the patient. By an intact FGF-23 gene it is meant that there are no alterations in the gene such as point mutations, deletions, insertions, chromosomal breakage, chromosomal rearrangements and the like wherein such alteration might alter production of FGF-23 or alter its biological activity, stability or the like to lead to disease processes or susceptibility to cellular degenerative conditions. Thus, in one embodiment of the present invention a method is provided for detecting and characterizing any alterations in the FGF-23 gene. The method comprises providing an oligonucleotide that contains the FGF-23 cDNA, genomic DNA or a fragment thereof or a derivative thereof. By a derivative of an oligonucleotide, it is meant that the derived oligonucleotide is substantially the same as the sequence from which it is derived in that the derived sequence has sufficient sequence complementarily to the sequence from which it is derived to hybridize to the FGF-23 gene. The derived nucleotide sequence is not necessarily physically derived from the nucleotide sequence, but may be generated in any manner including for example, chemical synthesis or DNA replication or reverse transcription or transcription.

Typically, patient genomic DNA is isolated from a cell sample from the patient and digested with one or more restriction endonucleases such as, for example, TaqI and AluI. Using the Southern blot protocol, which is well known in the art, this assay determines whether a patient or a particular tissue in a patient has an intact FGF-23 gene or an FGF-23 gene abnormality.

Hybridization to an FGF-23 gene would involve denaturing the chromosomal DNA to obtain a single-stranded DNA; contacting the single-stranded DNA with a gene probe associated with the FGF-23 gene sequence; and identifying the hybridized DNA-probe to detect chromosomal DNA containing at least a portion of a human FGF-23 gene.

The term "probe" as used herein refers to a structure comprised of a polynucleotide that forms a hybrid structure with a target sequence, due to complementarity of probe sequence with a sequence in the target region. Oligomers suitable for use as probes may contain a minimum of about 8-12 contiguous nucleotides which are complementary to the targeted sequence and preferably a minimum of about 20.

The FGF-23 gene probes of the present invention can be DNA or RNA oligonucleotides and can be made by any method known in the art such as, for example, excision, transcription or chemical synthesis. Probes may be labeled with any detectable label known in the art such as, for example, radioactive or fluorescent labels or enzymatic marker. Labeling of the probe can be accomplished by any method known in the art such as by PCR, random priming, end labeling, nick translation or the like. One skilled in the art will also recognize that other methods not employing a labeled probe can be used to determine the hybridization. Examples of methods that can be used for detecting hybridization include Southern blotting, fluorescence in situ hybridization, and single-strand conformation polymorphism with PCR amplification.

Hybridization is typically carried out at 25° - 45° C, more preferably at 32° - 40° C and more preferably at 37° - 38° C. The time required for hybridization is from about 0.25 to about 96 hours, more preferably from about one to about 72 hours, and most preferably from about 4 to about 24 hours.

FGF-23 gene abnormalities can also be detected by using the PCR method and primers that flank or lie within the FGF-23 gene. The PCR method is well known in the art. Briefly, this method is performed using two oligonucleotide primers which are capable of hybridizing to the nucleic acid sequences flanking a target sequence that lies within an FGF-23 gene and amplifying the target sequence. The terms "oligonucleotide primer" as used herein refers to a short strand of DNA or RNA ranging in length from about 8 to about 30 bases. Examples of oligonucleotide primers useful for this purpose are SEQ ID NO:5 and SEQ ID NO:6. The upstream and downstream primers are typically from about 20 to about 30 base pairs in length and hybridize to the flanking regions for replication of the nucleotide sequence. The polymerization is catalyzed by a DNA-polymerase in the presence of deoxynucleotide triphosphates or nucleotide analogs to produce double-stranded DNA molecules. The double strands are then separated by any denaturing method including physical, chemical or enzymatic. Commonly, the method of physical denaturation is used involving heating the nucleic acid, typically to temperatures from about 80°C. to 105°C for times ranging from about 1 to about 10 minutes. The process is repeated for the desired number of cycles.

The primers are selected to be substantially complementary to the strand of DNA being amplified. Therefore, the primers need not reflect the exact sequence of the template, but must be sufficiently complementary to selectively hybridize with the strand being amplified.

After PCR amplification, the DNA sequence comprising FGF-23 or pre-pro FGF-23 or a fragment thereof is then directly sequenced and analyzed by comparison of the sequence with the sequences disclosed herein to identify alterations which might change activity or expression levels or the like.

In another embodiment, a method for detecting FGF-23 is provided based upon an analysis of tissue expressing the FGF-23 gene. The method comprises hybridizing a polynucleotide to mRNA from a sample of tissues that normally express the FGF-23 gene. The sample is obtained from a patient suspected of having an abnormality in the FGF-23 gene or in the FGF-23 gene of particular cells.

To detect the presence of mRNA encoding FGF-23 protein, a sample is obtained from a patient. The sample can be from blood or from a tissue biopsy sample. The sample may be treated to extract the nucleic acids contained therein. The resulting nucleic acid from the sample is subjected to gel electrophoresis or other size separation techniques.

The mRNA of the sample is contacted with a DNA sequence serving as a probe to form hybrid duplexes. The use of a labeled probes as discussed above allows detection of the resulting duplex.

When using the cDNA encoding FGF-23 protein or a derivative of the cDNA as a probe, high stringency conditions can be used in order to prevent false positives, that is the hybridization and apparent detection of FGF-23 nucleotide sequences when in fact an intact and functioning FGF-23 gene is not present. When using sequences derived from the FGF-23 cDNA, less stringent conditions could be used, however, this would be a less preferred approach because of the likelihood of false positives. The stringency of hybridization is determined by a number of factors during hybridization and during the washing procedure, including temperature, ionic strength, length of time and concentration of formamide. These factors are outlined in, for example, Sambrook et al., Molecular Cloning: A Laboratory Manual, 2nd Ed. (1989) Cold Spring Harbor Press, Cold Spring Harbor, NY.

In order to increase the sensitivity of the detection in a sample of mRNA encoding the FGF-23 protein, the technique of reverse transcription/polymerization chain reaction (RT/PCR) can be used to amplify cDNA transcribed from mRNA encoding the FGF-23 protein. The method of RT/PCR is well known in the art, and can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and FGF-23 specific primers. (Belyavsky et al., Nucl. Acid Res. 17:2919-2932, 1989; Krug and Berger, Methods in Enzymology, 152:316-325, Academic Press, NY, 1987 which are incorporated by reference).

The polymerase chain reaction method is performed as described above using two oligonucleotide primers that are substantially complementary to the two flanking regions of the DNA segment to be amplified.

Following amplification, the PCR product is then electrophoresed and detected by ethidium bromide staining or by phosphoimaging.

The present invention further provides for methods to detect the presence of the FGF-23 protein in a sample obtained from a patient. Any method known in the art for detecting proteins can be used. Such methods include, but are not limited to immunodiffusion, immunoelectrophoresis, immunochemical methods, binder-ligand assays, immunohistochemical techniques, agglutination and complement assays. (for example, *see* Basic and Clinical Immunology, 217-262, Sites and Terr, eds., Appleton & Lange, Norwalk, CT, 1991 which is incorporated by reference). Preferred are binder-ligand immunoassay methods including reacting antibodies with an epitope or epitopes of the FGF-23 protein and competitively displacing a labeled FGF-23 protein or derivative thereof. Preferred antibodies are prepared according to Example 3.

As used herein, a derivative of the FGF-23 protein is intended to include a polypeptide in which certain amino acids have been deleted or replaced or changed to modified or unusual amino acids wherein the FGF-23 derivative is biologically equivalent to FGF-23 and wherein the polypeptide derivative cross-reacts with antibodies raised against the FGF-23 protein. By cross-reaction it is meant that an antibody reacts with an antigen other than the one that induced its formation.

Numerous competitive and non-competitive protein binding immunoassays are well known in the art. Antibodies employed in such assays may be unlabeled, for example as used in agglutination tests, or labeled for use in a wide variety of assay methods. Labels that can be used include radionuclides, enzymes, fluorescers, chemiluminescers, enzyme substrates or co-factors, enzyme inhibitors, particles, dyes and the like for use in radioimmunoassay (RIA), enzyme immunoassays, e.g., enzyme-linked immunosorbent assay (ELISA), fluorescent immunoassays and the like.

Polyclonal or monoclonal antibodies to the FGF-23 protein or an epitope thereof can be made for use in immunoassays by any of a number of methods known in the art. By epitope reference is made to an antigenic determinant of a polypeptide. An epitope could comprise 3 amino acids in a spatial conformation which is unique to the epitope. Generally an epitope consists of at least 5 such amino acids. Methods of determining the spatial conformation of amino acids are known in the art, and include, for example, x-ray crystallography and 2 dimensional nuclear magnetic resonance.

One approach for preparing antibodies to a protein is the selection and preparation of an amino acid sequence of all or part of the protein, chemically synthesizing the sequence and injecting it into an appropriate animal, usually a rabbit or a mouse (*see* Example 4).

Oligopeptides can be selected as candidates for the production of an antibody to the FGF-23 protein based upon the oligopeptides lying in hydrophilic regions, which are thus likely to be exposed in the mature protein. Preferred oligopeptides are RRHTRSAEDDSERD (residues 175-189 of SEQ ID NO:4) and YHLQIHKNGHVDGAPHQ (residues 51-67 of SEQ ID NO:4). Additional oligopeptides can be determined using, for example, the Antigenicity Index of Welling, G.W. et al., FEBS Lett. 188:215-218, 1985, incorporated herein by reference.

Other antibodies can be raised against a cleavage product of FGF-23 as disclosed herein, such as the ∼20 kDa and ∼7-12 kDa fragments of FGF-23.

Antibodies to FGF-23 can also be raised against oligopeptides that include one or more of the conserved regions identified herein such that the antibody can cross-react with other family members. Such antibodies can be used to identify and isolate the other family members.

Methods for preparation of the FGF-23 protein or an epitope thereof include, but are not limited to chemical synthesis, recombinant DNA techniques or isolation from biological samples. Chemical synthesis of a peptide can be performed, for example, by the classical Merrifeld method of solid phase peptide synthesis (Merrifeld, J. Am. Chem. Soc. 85:2149, 1963 which is incorporated by reference) or the FMOC strategy on a Rapid Automated Multiple Peptide Synthesis system (E. I. du Pont de Nemours Company, Wilmington, DE) (Caprino and Han, J. Org. Chem. 37:3404, 1972 which is incorporated by reference).

Polyclonal antibodies can be prepared by immunizing rabbits or other animals by injecting antigen followed by subsequent boosts at appropriate intervals. The animals are bled and sera assayed against purified FGF-23 protein usually by ELISA or by bioassay based upon the ability to block the action of FGF-23 on liver or other cells. When using avian species, e.g., chicken, turkey and the like, the antibody can be isolated from the yolk of the egg. Monoclonal antibodies can be prepared after the method of Milstein and Kohler by fusing splenocytes from immunized mice with continuously replicating tumor cells such as myeloma or lymphoma cells. (Milstein and Kohler, Nature 256:495-497, 1975; Gulfre and Milstein, Methods in Enzymology: Immunochemical Techniques 73:1-46, Langone and Banatis eds., Academic Press, 1981 which are incorporated by reference). The hybridoma cells so formed are then cloned by limiting dilution methods and supernates assayed for antibody production by ELISA, RIA or bioassay.

The unique ability of antibodies to recognize and specifically bind to target proteins provides an approach for treating an overexpression of the protein. Thus, another aspect of the present invention provides for a method for preventing or treating diseases involving overexpression of the FGF-23 protein by treatment of a patient with specific antibodies to the FGF-23 protein.

Specific antibodies, either polyclonal or monoclonal, to the FGF-23 protein can be produced by any suitable method known in the art as discussed above. For example, murine or human monoclonal antibodies can be produced by hybridoma technology or, alternatively, the FGF-23 protein, or an immunologically active fragment thereof, or an anti-idiotypic antibody, or fragment thereof can be administered to an animal to elicit the production of antibodies capable of recognizing and binding to the FGF-23 protein. Such antibodies can be from any class of antibodies including, but not limited to IgG, IgA, IgM, IgD, and IgE or in the case of avian species, IgY and from any subclass of antibodies.

Polypeptides encoded by the instant polynucleotides and corresponding full-length genes can be used to screen peptide libraries, protein libraries, small molecule libraries, and phage display libraries, and other known methods, to identify analogs or antagonists.

Native FGF polypeptides may play a role in cancer. For example, FGF family members can induce marked morphological transformation of NIH 3T3 cells, and exhibit strong tumorigenicity in nude mice. Angiogenic activity has been exhibited by FGF family members. Thus, inhibitors of FGF can be used to treat cancer, such as prostate cancer.

A library of peptides may be synthesized following the methods disclosed in U.S. Patent No. 5,010,175, and in PCT No. WO 91/17823. As described below in brief, a mixture of peptides is prepared, which is then screened to identify the peptides exhibiting the desired signal transduction and receptor binding activity. According to the method of the '175 patent, a suitable peptide synthesis support (e.g., a resin) is coupled to a mixture of appropriately protected, activated amino acids. The concentration of each amino acid in the reaction mixture is balanced or adjusted in inverse proportion to its coupling reaction rate so that the product is an equimolar mixture of amino acids coupled to the starting resin. The bound amino acids are then deprotected, and reacted with another balanced amino acid mixture to form an equimolar mixture of all possible dipeptides. This process is repeated until a mixture of peptides of the desired length (e.g., hexamers) is formed. Note that one need not include all amino acids in each step: one may include only one or two amino acids in some steps (e.g., where it is known that a particular amino acid is essential in a given position), thus reducing the complexity of the mixture. After the synthesis of the peptide library is completed, the mixture of peptides is screened for binding to the selected polypeptide. The peptides are then tested for their ability to inhibit or enhance activity. Peptides exhibiting the desired activity are then isolated and sequenced.

The method described in PCT No. WO 91/17823 is similar. However, instead of reacting the synthesis resin with a mixture of activated amino acids, the resin is divided into twenty equal portions (or into a number of portions corresponding to the number of different amino acids to be added in that step), and each amino acid is coupled individually to its portion of resin. The resin portions are then combined, mixed, and again divided into a number of equal portions for reaction with the second amino acid. In this manner, each reaction may be easily driven to completion. Additionally, one may maintain separate "subpools" by treating portions in parallel, rather than combining all resins at each step. This simplifies the process of determining which peptides are responsible for any observed receptor binding or signal transduction activity.

In such cases, the subpools containing, e.g., 1-2,000 candidates each are exposed to one or more polypeptides of the invention. Each subpool that produces a positive result is then resynthesized as a group of smaller subpools (sub-subpools) containing, e.g., 20-100 candidates, and reassayed. Positive sub-subpools may be resynthesized as individual compounds, and assayed finally to determine the peptides that exhibit a high binding constant. These peptides can be tested for their ability to inhibit or enhance the native activity. The methods described in PCT No. WO 91/7823 and U.S. Patent No. 5,194,392 (herein incorporated by reference) enable the preparation of such pools and subpools by automated techniques in parallel, such that all synthesis and resynthesis may be performed in a matter of days.

Peptide agonists or antagonists are screened using any available method, such as signal transduction, antibody binding, receptor binding and mitogenic assays. The assay conditions ideally should resemble the conditions under which the native activity is exhibited *in vivo*, that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide may require concentrations equal to or greater than the native concentration, while inhibitors capable of binding irreversibly to the polypeptide may be added in concentrations on the order of the native concentration.

The availability of hFGF-23 and mFGF-23 allows for the identification of small molecules and low molecular weight compounds that inhibit the binding of FGF-23 to its receptor, through routine application of high-throughput screening methods (HTS). HTS methods generally refer to technologies that permit the rapid assaying of lead compounds for therapeutic potential. HTS techniques employ robotic handling of test materials, detection of positive signals, and interpretation of data. Lead compounds may be identified via the incorporation of radioactivity or through optical assays that rely on absorbance, fluorescence or luminescence as read-outs. Gonzalez, J.E. et al., (1998) Curr. Opin. Biotech. 9:624-631. Assays for detecting interaction between an FGF molecule and FGF receptor are described in, for example, Blunt, A. G. et al., (1997) J. Biol. Chem. 272:3733-3738, and such assays can be adapted for determining if a candidate molecule can inhibit the interaction between FGF-23 and its receptor.

Model systems are available that can be adapted for use in high throughput screening for compounds that inhibit the interaction of FGF-23 with its receptor, for example by competing with FGF-23 for receptor binding. Sarubbi et al., (1996) Anal. Biochem. 237:70-75 describe cell-free, non-isotopic assays for identifying molecules that compete with natural ligands for binding to the active site of IL-1 receptor. Martens, C. et al., (1999) Anal. Biochem. 273:20-31 describe a generic particle-based nonradioactive method in which a labeled ligand binds to its receptor immobilized on a particle; label on the particle decreases in the presence of a molecule that competes with the labeled ligand for receptor binding.

The therapeutic FGF-23 polynucleotides and polypeptides of the present invention may be utilized in gene delivery vehicles. The gene delivery vehicle may be of viral or non-viral origin (*see generally*, Jolly, Cancer Gene Therapy 1:51-64 (1994); Kimura, Human Gene Therapy 5:845-852 (1994); Connelly, Human Gene Therapy 1:185-193 (1995); and Kaplitt, Nature Genetics 6:148-153 (1994)). Gene therapy vehicles for delivery of constructs including a coding sequence of a therapeutic of the invention can be administered either locally or systemically. These constructs can utilize viral or non-viral vector approaches. Expression of such coding sequences can be induced using endogenous mammalian or heterologous promoters. Expression of the coding sequence can be either constitutive or regulated.

The present invention can employ recombinant retroviruses which are constructed to carry or express a selected nucleic acid molecule of interest. Retrovirus vectors that can be employed include those described in EP 0 415 731; WO 90/07936; WO 94/03622; WO 93/25698; WO 93/25234; U.S. Patent No. 5,219,740; WO 93/11230; WO 93/10218; Vile and Hart, Cancer Res. 53:3860-3864 (1993); Vile and Hart, Cancer Res. 53:962-967 (1993); Ram et al., Cancer Res. 53:83-88 (1993); Takamiya et al., J. Neurosci. Res. 33:493-503 (1992); Baba et al., J. Neurosurg. 79:729-735 (1993); U.S. Patent No. 4,777,127; GB Patent No. 2,200,651; and EP 0 345 242. Preferred recombinant retroviruses include those described in WO 91/02805.

Packaging cell lines suitable for use with the above-described retroviral vector constructs may be readily prepared (*see* PCT publications WO 95/30763 and WO 92/05266), and used to create producer cell lines (also termed vector cell lines) for the production of recombinant vector particles. Within particularly preferred embodiments of the invention, packaging cell lines are made from human (such as HT1080 cells) or mink parent cell lines, thereby allowing production of recombinant retroviruses that can survive inactivation in human serum.

The present invention also employs alphavirus-based vectors that can function as gene delivery vehicles. Such vectors can be constructed from a wide variety of alphaviruses, including, for example, Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532). Representative examples of such vector systems include those described in U.S. Patent Nos. 5,091,309; 5,217,879; and 5,185,440; and PCT Publication Nos. WO 92/10578; WO 94/21792; WO 95/27069; WO 95/27044; and WO 95/07994.

Gene delivery vehicles of the present invention can also employ parvovirus such as adeno-associated virus (AAV) vectors. Representative examples include the AAV vectors disclosed by Srivastava in WO 93/09239, Samulski et al., J. Vir. 63:3822-3828 (1989); Mendelson et al., Virol. 166:154-165 (1988); and Flotte et al., P.N.A.S. 90:10613-10617 (1993).

Representative examples of adenoviral vectors include those described by Berkner, Biotechniques 6:616-627 (Biotechniques); Rosenfeld et al., Science 252:431-434 (1991); WO 93/19191; Kolls et al., P.N.A.S. :215-219 (1994); Kass-Eisler et al., P.N.A.S. 90:11498-11502 (1993); Guzman et al., Circulation 88:2838-2848 (1993); Guzman et al., Cir. Res. 73:1202-1207 (1993); Zabner et al., Cell 75:207-216 (1993); Li et al., Hum. Gene Ther. 4:403-409 (1993); Cailaud et al., Eur. J. Neurosci. 5:1287-1291 (1993); Vincent et al., Nat. Genet. 5:130-134 (1993); Jaffe et al., Nat. Genet. 1:372-378 (1992); and Levrero et al., Gene 101:195-202 (1992). Exemplary adenoviral gene therapy vectors employable in this invention also include those described in WO 94/12649, WO 93/03769; WO 93/19191; WO 94/28938; WO 95/11984 and WO 95/00655. Administration of DNA linked to killed adenovirus as described in Curiel, Hum. Gene Ther. 3:147-154 (1992) may be employed.

Other gene delivery vehicles and methods may be employed, including polycationic condensed DNA linked or unlinked to killed adenovirus alone, for example Curiel, Hum. Gene Ther. 3:147-154 (1992); ligand-linked DNA, for example *see* Wu, J. Biol. Chem. 264:16985-16987 (1989); eukaryotic cell delivery vehicles cells, for example *see* U.S. Serial No. 08/240,030, filed May 9, 1994, and U.S. Patent No. 6,015,686; deposition of photopolymerized hydrogel materials; hand-held gene transfer particle gun, as described in U.S. Patent No. 5,149,655; ionizing radiation as described in U.S. Patent No. 5,206,152 and in WO 92/11033; nucleic charge neutralization or fusion with cell membranes. Additional approaches are described in Philip, Mol. Cell Biol. 14:2411-2418 (1994), and in Woffendin, Proc. Natl. Acad. Sci. 91:1581-1585 (1994).

Naked DNA may also be employed. Exemplary naked DNA introduction methods are described in WO 90/11092 and U.S. Patent No. 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm. Liposomes that can act as gene delivery vehicles are described in U.S. Patent No. 5,422,120, PCT Patent Publication Nos. WO 95/13796, WO 94/23697, and WO 91/14445, and EP No. 0 524 968.

Further non-viral delivery suitable for use includes mechanical delivery systems such as the approach described in Woffendin et al., Proc. Natl. Acad. Sci. USA 91(24):11581-11585 (1994). Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in U.S. Patent No. 5,149,655; use of ionizing radiation for activating transferred gene, as described in U.S. Patent No. 5,206,152 and PCT Patent Publication No. WO 92/11033.

FGF has been implicated in diseases characterized by loss of function, inadequate function/number, abnormal function or death of cells, tissues or organs for which function or survival can be prolonged/rescued, and abnormalities reversed or prevented by therapy with FGF.

Loss of pulmonary, bronchia or alveolar cells or function, healing of pulmonary or bronchia wounds, pulmonary infraction, emphysema/chronic obstructive pulmonary disease, asthma, sequelae of infectious or autoimmune disease, sequelae of pulmonary arterial or venous hypertension, pulmonary fibrosis, pulmonary disease of immaturity, and cystic fibrosis are conditions amenable to treatment with FGF.

Ischemic vascular disease may be amenable to FGF-23 treatment, wherein the disease is characterized by inadequate blood flow to an organ(s). Treatment may induce therapeutic angiogenesis or preserve function/survival of cells (myocardial ischemia/infarction, peripheral vascular disease, renal artery disease, stroke). Cardiomyopathies characterized by loss of function or death of cardiac myocytes or supporting cells in the heart (congestive heart failure, myocarditis, heart failure, arrhythmias, valvular disorders, hypertrophy, congenital defects, cardiac or coronary arterial injuries, wounds, inflammation and surgical trauma) may also be treated using FGF-23, as can musculoskeletal disease characterized by loss of function, inadequate function or death of skeletal muscle cells, bone cells or supporting cells. Examples include skeletal myopathies, bone disease, and arthritis. FGF-23 polynucleotides and polypeptides may aid in correction of congenital defects due to loss of FGF-23 molecule or its function (liver, heart, lung, brain, limbs, kidney, etc.).

Treatment of wound healing is yet another use of FGF-23 polypeptides and polynucleotides, either due to trauma, disease, medical or surgical treatment, including regeneration of cell populations and tissues depleted by these processes. Examples include liver regeneration, operative wound healing, re-endothelialization of injured blood vessels, healing of traumatic wounds, healing of ulcers due to vascular, metabolic disease, etc., bone fractures, loss of cells due to inflammatory disease, etc.

FGF-23 may also be used in screens to identify drugs for treatment of cancers which involve over activity of the molecule, or new targets which would be useful in the identification of new drugs.

For all of the preceding embodiments, the clinician will determine, based on the specific condition, whether FGF-23 polypeptides or polynucleotides, antibodies to FGF-23, or small molecules such as peptide analogues or antagonists, will be the most suitable form of treatment. These forms are all within the scope of the invention.

Preferred embodiments of the invention are described in the following examples. Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims which follow the examples.

### EXAMPLES

### EXAMPLE 1

*Isolation and Analysis of Mouse FGF-23.* DNA was amplified from mouse skin cDNA by adaptor-ligation mediated PCR using a Marathon cDNA amplification kit (Clontech) with the following primers: 5' ctgatgattacatcagaggac 3' (a sense primer for mouse FGF-23, SEQ ID NO:7); 5' caccaggtagtgatgcttct 3' (an antisense primer for mouse FGF-23, SEQ II7 NO:8); 5' atccatacaaaggaaccttcg 3' (an antisense primer for mouse FGF-23, SEQ ID NO:9); 5' ccatcctaatacgactcactatagggc 3' (an adaptor primer, SEQ ID NO:10); and 5' actcactatagggctcgagcggc 3' (an adaptor primer, SEQ ID NO:11). The cDNA encoding the entire coding region of the FGF was amplified by PCR using the primers 5' actcagtgctgtgcaatgct 3' (a sense primer for mouse FGF-23) and 5' gacctagacgaacctgggaa 3' (an antisense primer for mouse FGF-23), and cloned into the pGEM-T DNA vector. The nucleotide sequence is shown in SEQ ID NO: 1 and the amino acid sequence is shown in SEQ ID NO:2. The protein has 251 amino acids, including a signal sequence at approximately amino acid positions 1-24.

### EXAMPLE 2

### Production of Recombinant Mouse FGF-23 in High Five Insect Cells.

The mouse FGF-23 cDNA with a DNA fragment (75 bp) encoding an E tag (GAPVPYPDPLEPR) and a His₆ tag (HHHHHH) at the 3'-terminus of the coding region was constructed in a transfer vector DNA, *pBacPAK9* (Clontech). Recombinant baculovirus containing the FGF-23 cDNA with the tag sequences was obtained by cotransfection of Sf9 cells with the recombinant *pBacPAK9* and a Bsu36 I-digested expression vector, *BacPAK6* (Clontech). High Five insect cells were infected with the resultant recombinant baculovirus and incubated at 27°C for 72 h in EX-CELL 400 COMPLETE medium (JRH Biosciences).

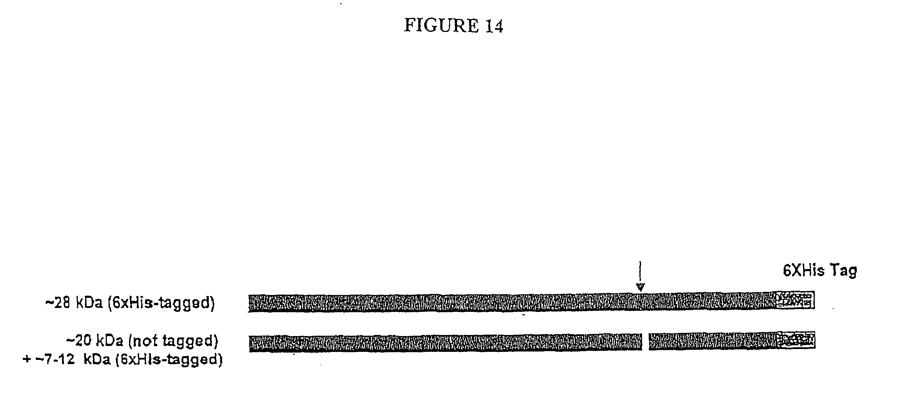

During expression, FGF-23 can undergo cleavage, yielding a ∼20 kDa fragment and a ∼7-12 kDa fragment, as shown schematically in Figure 14. The sizes of the fragments are consistent with proteolytic cleavage during processing, near the residues 176 and 179, both of which are arginines.

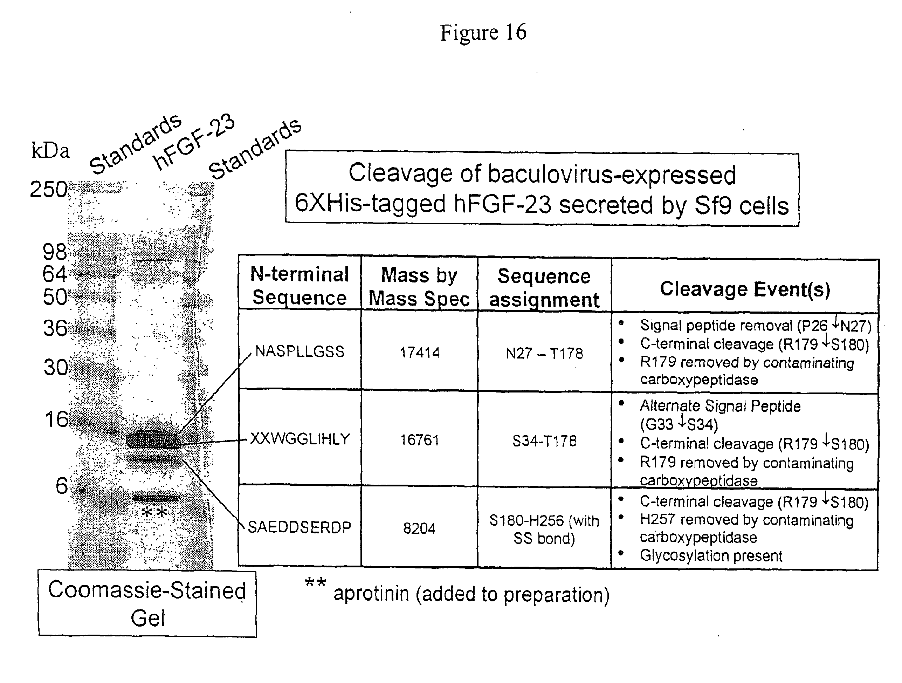

Cleavage between positions 179 and 180 was confirmed by analyzing the baculovirus-expressed protein purified from SF9 cells, as shown in Figure 16. The cell media contained several fragments of hFGF-23. Two fragments represent near-full length forms in which the signal peptide is removed from the N-terminus, consistent with a secreted molecule. The signal peptide is removed by cleavage between P26 and N27. An alternate signal peptide cleavage site is between G33 and S34, yielding a slightly smaller molecule. For both of these variants, cleavage can occur at the R179 cleavage site. The media also contained a C-terminal fragment consisting of 5180-H256. The three cleavage products are shown in Table 1 below, and correlate with the molecular weight bands at 17414, 16761, and 8204 in Figure 16.

**TABLE 1**

| **N-terminal Sequence** | **Mass by Mass Spec** | **Sequence assignment** | **Cleavage Events** |
|---|---|---|---|
| NASPLLGSS | 17414 | N27 - T178 | • Signal peptide removal (P26↓N27) |
| | | | • C-terminal cleavage (R179↓S180) |
| | | | • R179 removal by contaminating carboxypeptidase |
| XXWGGLIHLY | 16761 | S34 - T178 | • Alternate Signal Peptide (G33↓S34) |
| | | | • C-terminal cleavage ((R179↓S180) |
| | | | • R179 removal by contaminating carboxypeptidase |
| SAEDDSERDP | 8204 | S180 - H256 (with SS bond) | • C-terminal cleavage ((R179↓S180) |
| | | | • H257 removed by contaminating carboxypeptidase |
| | | | • Glycosylation present |

### EXAMPLE 3

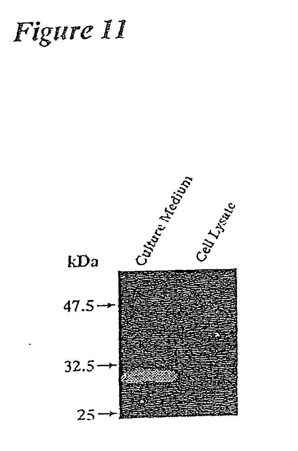

*Detection of Recombinant GF-23 by Western Blotting Analysis.* The culture medium and cell lysate of High Five cells infected with the recombinant baculovirus were separated by sodium dodecyl sulfate (SDS)-polyacrylamide gel (12.5%) electrophoresis under reducing conditions and transferred onto a nitrocellulose membrane (Hybond-ECL, Amersham Pharmacia Biotech). The membrane was incubated with anti-E tag antibodies (1:500) (Amersham Pharmacia Biotech). The protein with the E tag was visualized as described (Hoshikawa et al., Biochem. Biophys. Res. Commun. 244:187-191 (1998)). The results are shown in Figure 11.

### EXAMPLE 4

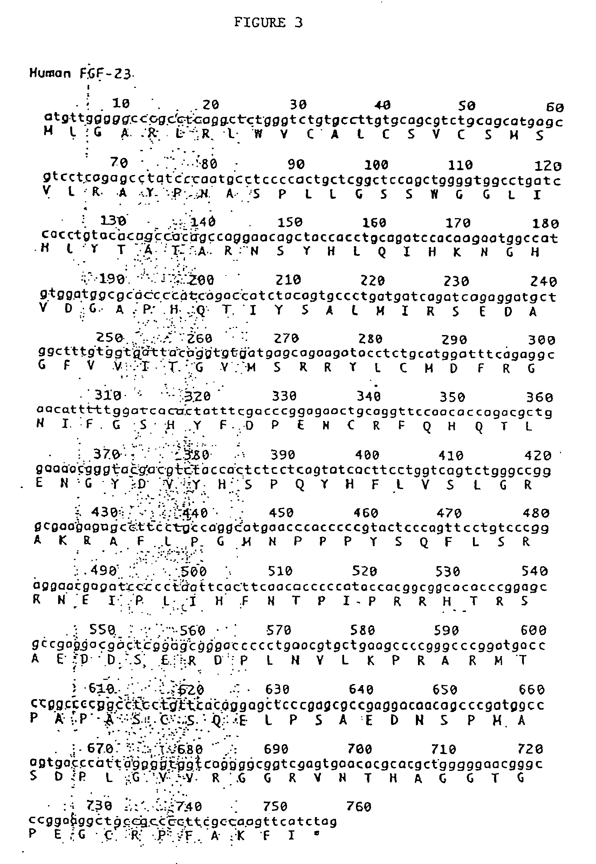

*Isolation and Analysis of Human FGF-23.* The human FGF-23 gene was located in chromosome 12p13. The protein contains 251 amino acids, as shown in SEQ ID NO:4 (Figure 3), and is encoded by the polynucleotide sequence of SEQ ID NO:3. Primers for amplification of human FGF-23 cDNA coding region are: sense primer: 5' agcaccagccactcagagca 3' (SEQ ID NO:5); antisense primer: 5' cttccagcgaccctagatga 3' (SEQ ID NO:6). The protein has a signal peptide at approximately amino acid positions 1-24.

### EXAMPLE 5

### Quantitative Analysis of FGF-23 mRNA Expression in Mouse Tissues by

*Real-Time Quantitative PCR.* Mouse tissue cDNA was synthesized in a reaction mixture (20 µl) containing Moloney murine leukemia virus reverse transcriptase, a random hexadeoxynucleotide primer and mouse tissue RNA (5 µg, OriGene) as a template. Mouse FGF-23 cDNA was amplified from the cDNA in a Model 7700 Sequence Detector (PE Applied Biosystem) with a forward primer, a reverse primer and a TaqMan probe specific for mouse FGF-23 cDNA. Mouse β-actin cDNA was also amplified with a forward primer, a reverse primer and a TaqMan probe specific mouse β-actin. Tokunaga, et al., Nucleic Acids Res. 14:2829 (1994)). The copy numbers of FGF-23 and β-actin cDNAs were determined according to the manufacturer's instructions. The results are shown in Figure 12. Of the tissues tested (brain, thymus, small intestine, heart, lung, liver, kidney, muscle, skin, spleen, stomach, and testis), FGF-23 mRNA was found to be mainly expressed in the brain and thymus. The results are summarized in Table 2.

**Table 2**

| **Tissue** | **Copy number ratio, FGF-23/**β **actin** |
|---|---|
| Brain | 1.5 ± 0.25 |
| Thymus | 1.45 ± 0.1 |
| Small intestine | 0.5 ± 0.1 |
| Heart | 0.25 ± 0.05 |

### EXAMPLE 6

*In Situ Hybridization.* Adult mouse brain and thymus were frozen in powdered dry ice, and sections were cut at 16 µm with a cryostate, thaw-mounted onto poly-L-lysine-coated slides, and stored at -85°C until hybridization. An ³⁵S-labeled mouse FGF-23 antisense or sense probe was transcribed using SP6 RNA polymerase or T7 RNA polymerase (TaKaRa) with uridine 5'-α-[³⁵S]thiotriphosphate (∼30TBq/mmol) (Amersham Pharmacia Biotech), respectively. The sections were examined by *in situ* hybridization with the labeled probe as described. (Yamasaki et al., J. Biol. Chem. 271:15918-15921 (1996)). As shown in Figure 13, label was detected in the ventrolateral thalamic nucleus (arrow, Figure 13).

### EXAMPLE 7

*Preparation of Antisera to FGF-23 by Immunization of Rabbits with an FGF-23 Peptide.* A peptide sequence corresponding to selected contiguous amino acids of the human FGF-23 protein is synthesized and coupled to keyhole limpet hemocyanin (KLH) as described (Harlow and Land, Antibodies: A Laboratory Manual, 1988. Cold Spring Harbor Laboratory, New York, NY). The KLH-coupled peptide is used to immunize rabbits. Antisera are tested for specificity to FGF-23, and for cross-reactivity with other FGF proteins.

Exemplary peptide sequences are: RRHTRSAEDDSERD (residues 175-189 of SEQ ID NO:4) and YHLQIHKNGHVDGAPHQ (residues 51-67 of SEQ ID NO:4).

### EXAMPLE 8

*Phosphate Uptake Assay in OK Cells.* The effects of FGF-23 and mutants or fragments thereof on phosphate uptake by opossum kidney cells is assayed as described in this example. The following reagents are used:

| | |
|---|---|
| FGF-23 | |
| FGF-2 (diluted to 1 nM in PBS) | |
| PTH (Parathyroid Hormone, Sigma, #P3796) Stock 100 ug/ml in PBS. | |
| Diluted to 1 ug/ml stock in PBS for experiment | |
| | |
| [³²P]orthophosphoric acid, NEN #NEX053 1 mCi/1 ml water. Dilute 1:10 i | |
| PBS for experiment | |
| Transferrin | |
| Insulin | |
| Uptake Solution: | 137 mM NaCl |
| | 5.4 mM KCl |
| | 1.8 mM CaCl2 |
| | 1.2 mM MgS04 |
| | 14 mM HEPES, pH 7.4 |
| | |
| Wash Solution (4°C): | 137 mM NaCl |
| | 14 mM HEPES, pH 7.4 |
| | |
| Extract Solution: | 0.5M NaOH |
| | 0.1% Triton X-100 |
| | |
| Neutralization Solution: | 0.5M HCl |

### The method is carried out as follows:

The cells are maintained in DMEM, 30 ug/ml transferrin, 5 ug/ml insulin, 5% FBS and Pen/Strep. To perform the assay, 24-well plates are seeded at 60,000 cells/well, and grown to confluence over 6-8 days, changing media every 3-4 days. On the day prior to assay, the media is changed to DMEM, transferrin, insulin, and 1 mg/ml BSA, with washes, 1 ml/well.
On the day of assay, the following steps are performed:
   (1) Treat Cells: Add Vehicle, FGF-23, FGF-2 or PTH as listed below. Incubate 3 hours at 37°C.
   (2) Wash Cells: 3 x 1-2 ml with Uptake Solution pre-warmed to 37°C; add 1 ml of fresh Uptake solution/well; place in incubator 10 minutes.
   (3) Label Cells: Add 20 ul (diluted stock solution = 2 uCi) of [³²P]orthophosphate per well, mix well by swirling, start timer. Return to incubator.
   (4) Terminate Uptake: At 15 minutes, terminate uptake by placing on ice, aspirating hot media, washing 4 x 1 ml *ice-cold* Wash Solution. Aspirate. Add 250 uL Extract Solution per well.
   (5) Extract Counts: After incubating Extracts 5-10 minutes, transfer to Eppendorf Centrifuge Tube. Wash well with additional 250 uL Extract Solution. Neutralize by adding 500 uL Neutralization Solution to Eppendorf Centrifuge Tube. Vortex to mix.
   (6) Count: Duplicate 100 uL aliquots per well in 5 ml scintillation fluid, ³²P channel.
   The treatments are as follows, with each treatment performed in quadruplicate.
Set 1: 100 uL/well Vehicle (PBS)/well.
Set 2: 100 uL/well PTH (1 ug/ml stock; 100 ng/ml final).
Set 3: 100 uL/well FGF-2 (1 nM stock in PBS; 100 pM final).
Set 4: 100 uL/well FGF-23 (the FGF-23 is full length, a cleavage product, and/or noncleavable FGF-23 mutant).
Set 5: Baculovirus control supe 6xHis column Flow-through, 100 uL/well.
Set 6: FGF-23 baculo 6xHis column Flow-through, 100 uL/well [cleaved form of FGF-23].

PTH should inhibit phosphate uptake in these cells (positive control).
FGF-2 may have no effect on phosphase uptake (negative control).
The phosphate uptake by cells treated with FGF-23 (including its cleavage products and noncleavable FGF-23 mutants) is compared with the cells treated with PTH and FGF-2, to determine the biological activity of the FGF-23.
   All patents, published patent applications and publications cited herein are incorporated by reference as if set forth fully herein.
   Although certain preferred embodiments have been described herein, it is not intended that such embodiments be construed as limitations on the scope of the invention except as set forth in the following claims.

## Claims

1. An isolated polypeptide comprising amino acids at least 95% identical to amino acids selected from the group consisting of:
(a) amino acids from about 1 to about 251 of SEQ ID NO : 4;
(b) amino acids from about 2 to about 251 of SEQ ID NO : 4;
(c) amino acids from about 1 to about 24 of SEQ ID NO : 4;
(d) amino acids from about 25 to about 251 of SEQ ID NO : 4;
(e) amino acids from about 1 to about 175 of SEQ ID NO : 4 ;
(f) amino acids from about 1 to about 177 of SEQ ID NO : 4;
(g) amino acids from about 177 to about 251 of SEQ ID NO : 4; and
(h) amino acids from about 180 to about 251 of SEQ ID NO : 4.

2. An isolated polypeptide wherein, except for at least one conservative amino acid substitution, said polypeptide has an amino acid sequence selected from the group consisting of:
(a) amino acids from about 1 to about 251 of SEQ ID NO : 4;
(b) amino acids from about 2 to about 251 of SEQ ID NO : 4:
(c) amino acids from about 1 to about 24 of SEQ ID NO : 4;
(d) amino acids from about 25 to about 251 of SEQ ID NO : 4;
(e) amino acids from about 1 to about 175 of SEQ ID NO : 4;
(f) amino acids from about 1 to about 177 of SEQ ID NO : 4;
(g) amino acids from about 177 to about 251 of SEQ ID NO : 4; and
(h) amino acids from about 180 to about 251 of SEQ ID NO : 4.

3. An isolated polypeptide comprising amino acids selected from the group consisting of:
(a) amino acids from about 1 to about 251 of SEQ ID NO : 4;
(b) amino acids from about 2 to about 251 of SEQ ID NO : 4;
(c) amino acids from about 1 to about 24 of SEQ ID NO : 4:
(d) amino acids from about 25 to about 251 of SEQ ID NO : 4;
(e) amino acids from about 1 to about 175 of SEQ ID NO : 4;
(f) amino acids from about 1 to about 177 of SEQ ID NO : 4;
(g) amino acids from about 177 to about 251 of SEQ ID NO : 4; and
(h) amino acids from about 180 to about 251 of SEQ ID NO : 4.

4. An isolated polypeptide as recited in SEQ ID NO:4.

5. The polypeptide of claim 4, further comprising an amino acid substitution or deletion at one or both of positions R176 and R179.

6. The polypeptide of claim 4, further comprising an amino acid substitution at position R179.

7. A fusion protein comprising a polypeptide according to any one of claims 1-6.

8. An isolated nucleic acid molecule encoding the polypeptide of any one of claims 1-7.

9. A recombinant vector comprising the nucleic acid of claim 8.

10. A recombinant host cell comprising the vector of claim 9.

11. A pharmaceutical composition comprising the polypeptide of any one of claims 1-7, in combination with a pharmaceutically acceptable carrier.

12. A polypeptide according to any one of claims 1-7 or a pharmaceutical composition according to claim 11 for use in therapy.

13. A polypeptide according to any one of claims 1-7 or a pharmaceutical composition according to claim 11 for use in the treatment of musculo-skeletal conditions including bone fractures, ligament and tissue repair, tendonitis, bursitis; skin conditions including, burns, cuts, lacerations, bed sores, slow healing ulcers; myocardial infarction and ischemia; neurological conditions including, neuro-degenerative disease and stroke; eye disease, including macular degeneration; abnormal proliferation, atrophy, degeneration, toxin-mediated tissue damage, degeneration of neuronal tissue, Parkinson's Disease and Alzheimer's Disease.

14. Use of a polypeptide according to any one of claims 1-7 or a pharmaceutical composition according to claim 11 in the manufacture of a medicament for the treatment of musculo-skeletal conditions including bone fractures, ligament and tissue repair, tendonitis, bursitis; skin conditions including, burns, cuts, lacerations, bed sores, slow healing ulcers; myocardial infarction and ischemia; neurological conditions including, neuro-degenerative disease and stroke; eye disease, including macular degeneration; abnormal proliferation, atrophy, degeneration, toxin-mediated tissue damage, degeneration of neuronal tissue, Parkinson's Disease and Alzheimer's Disease.


SEQUENCE LISTING

<110> Novartis Vaccines and Diagnostics, Inc.
      Kyoto University

<120> HUMAN FGF-23 GENE AND GENE EXPRESSION PRODUCTS

<130> N.83761B

<140> Divisional of EP 01922308.0
<141> 2001-03-07

<150> EP 01922308.0
<151> 2001-03-07

<150> PCT/US01/07468
<151> 2001-03-07

<150> US 60/251,650
<151> 2000-12-05

<150> US 60/233,368
<151> 2000-09-18

<150> US 60/187,854
<151> 2000-03-08

<160> 49

<170> FastSEQ for Windows Version 4.0

<210> 1
<211> 756
<212> DNA
<213> Mus musculus

<400> 1
atgctaggga cctgccttag actcctggtg ggcgtgctct gcactgtctg cagcttgggc      60
actgctagag cctatccaga cacttcccca ttgcttggct ccaactgggg aagcctgacc     120
cacctgtaca cggctacagc caggaccagc tatcacctac agatccatag ggatggtcat     180
gtagatggca ccccccatca gaccatctac agtgccctga tgattacatc agaggacgcc     240
ggctctgtgg tgataacagg agccatgact cgaaggttcc tttgtatgga tctccacggc     300
aacatttttg gatcgcttca cttcagccca gagaattgca agttccgcca gtggacgctg     360
gagaatggct atgacgtcta cttgtcgcag aagcatcact acctggtgag cctgggccgc     420
gccaagcgca ttttccagcc gggcaccaac ccgccgccct tctcccagtt cctggctcgc     480
aggaacgagg tcccgctgct gcacttctac actgttcgcc cacggcgcca cacgcgcagc     540
gccgaggacc cacccgagcg cgacccactg aacgtgctca agccgcggcc ccgcgccacg     600
cctgtgcctg tatcctgctc tcgcgagctg ccgagcgcag aggaaggtgg ccccgcagcc     660
agcgatcctc tgggggtgct gcgcagaggc cgtggagatg ctcgcggggg cgcgggaggc     720
gcggataggt gtcgcccctt tcccaggttc gtctag                               756

<210> 2
<211> 251
<212> PRT
<213> Mus musculus

<400> 2
Met Leu Gly Thr Cys Leu Arg Leu Leu Val Gly Val Leu Cys Thr Val
 1               5                   10                  15

Cys Ser Leu Gly Thr Ala Arg Ala Tyr Pro Asp Thr Ser Pro Leu Leu
            20                  25                  30
Gly Ser Asn Trp Gly Ser Leu Thr His Leu Tyr Thr Ala Thr Ala Arg
        35                  40                  45
Thr Ser Tyr His Leu Gln Ile His Arg Asp Gly His Val Asp Gly Thr
    50                  55                  60
Pro His Gln Thr Ile Tyr Ser Ala Leu Met Ile Thr Ser Glu Asp Ala
65                  70                  75                  80
Gly Ser Val Val Ile Thr Gly Ala Met Thr Arg Arg Phe Leu Cys Met
                85                  90                  95
Asp Leu His Gly Asn Ile Phe Gly Ser Leu His Phe Ser Pro Glu Asn
            100                 105                 110
Cys Lys Phe Arg Gln Trp Thr Leu Glu Asn Gly Tyr Asp Val Tyr Leu
        115                 120                 125
Ser Gln Lys His His Tyr Leu Val Ser Leu Gly Arg Ala Lys Arg Ile
    130                 135                 140
Phe Gln Pro Gly Thr Asn Pro Pro Pro Phe Ser Gln Phe Leu Ala Arg
145                 150                 155                 160
Arg Asn Glu Val Pro Leu Leu His Phe Tyr Thr Val Arg Pro Arg Arg
                165                 170                 175
His Thr Arg Ser Ala Glu Asp Pro Pro Glu Arg Asp Pro Leu Asn Val
            180                 185                 190
Leu Lys Pro Arg Pro Arg Ala Thr Pro Val Pro Val Ser Cys Ser Arg
        195                 200                 205
Glu Leu Pro Ser Ala Glu Glu Gly Gly Pro Ala Ala Ser Asp Pro Leu
    210                 215                 220
Gly Val Leu Arg Arg Gly Arg Gly Asp Ala Arg Gly Gly Ala Gly Gly
225                 230                 235                 240
Ala Asp Arg Cys Arg Pro Phe Pro Arg Phe Val
                245                 250

<210> 3
<211> 756
<212> DNA
<213> Homo sapiens

<400> 3
atgttggggg cccgcctcag gctctgggtc tgtgccttgt gcagcgtctg cagcatgagc      60
gtcctcagag cctatcccaa tgcctcccca ctgctcggct ccagctgggg tggcctgatc     120
cacctgtaca cagccacagc caggaacagc taccacctgc agatccacaa gaatggccat     180
gtggatggcg caccccatca gaccatctac agtgccctga tgatcagatc agaggatgct     240
ggctttgtgg tgattacagg tgtgatgagc agaagatacc tctgcatgga tttcagaggc     300
aacatttttg gatcacacta tttcgacccg gagaactgca ggttccaaca ccagacgctg     360
gaaaacgggt acgacgtcta ccactctcct cagtatcact tcctggtcag tctgggccgg     420
gcgaagagag ccttcctgcc aggcatgaac ccacccccgt actcccagtt cctgtcccgg     480
aggaacgaga tccccctaat tcacttcaac acccccatac cacggcggca cacccggagc     540
gccgaggacg actcggagcg ggaccccctg aacgtgctga agccccgggc ccggatgacc     600
ccggccccgg cctcctgttc acaggagctc ccgagcgccg aggacaacag cccgatggcc     660
agtgacccat taggggtggt caggggcggt cgagtgaaca cgcacgctgg gggaacgggc     720
ccggaaggct gccgcccctt cgccaagttc atctag                               756

<210> 4
<211> 251
<212> PRT
<213> Homo sapiens

<400> 4

<210> 5
<211> 20
<212> DNA
<213> Artificial Sequence

<220>
<223> Sense PCR primer

<400> 5
agcaccagcc actcagagca                                                20

<210> 6
<211> 20
<212> DNA
<213> Artificial Sequence

<220>
<223> Antisense PCR primer

<400> 6
cttccagcga ccctagatga                                                20

<210> 7
<211> 21
<212> DNA
<213> Artificial Sequence

<220>
<223> Sense primer for mouse FGF-23

<400> 7
ctgatgatta catcagagga c                                                21

<210> 8
<211> 20
<212> DNA
<213> Artificial Sequence

<220>
<223> Antisense primer for mouse FGF-23

<400> 8
caccaggtag tgatgcttct                                                  20

<210> 9
<211> 21
<212> DNA
<213> Artificial Sequence

<220>
<223> Antisense primer for mouse FGF-23

<400> 9
atccatacaa aggaaccttc g                                                21

<210> 10
<211> 27
<212> DNA
<213> Artificial Sequence

<220>
<223> adaptor primer

<400> 10
ccatcctaat acgactcact atagggc                                          27

<210> 11
<211> 23
<212> DNA
<213> Artificial Sequence

<220>
<223> adaptor primer

<400> 11
actcactata gggctcgagc ggc                                              23

<210> 12
<211> 20
<212> DNA
<213> Artificial Sequence

<220>
<223> Sense primer for mouse FGF-23.

<400> 12
actcagtgct gtgcaatgct                                                  20

<210> 13
<211> 20

<212> DNA
<213> Artificial Sequence

<220>
<223> Antisense primer for mouse FGF-23

<400> 13
gacctagacg aacctgggaa                                                    20

<210> 14
<211> 216
<212> PRT
<213> Homo sapiens

<400> 14
Met Arg Ser Gly Cys Val Val Val His Val Trp Ile Leu Ala Gly Leu
1               5                   10                  15
Trp Leu Ala Val Ala Gly Arg Pro Leu Ala Phe Ser Asp Ala Gly Pro
            20                  25                  30
His Val His Tyr Gly Trp Gly Asp Pro Ile Arg Leu Arg His Leu Tyr
        35                  40                  45
Thr Ser Gly Pro His Gly Leu Ser Ser Cys Phe Leu Arg Ile Arg Ala
    50                  55                  60
Asp Gly Val Val Asp Cys Ala Arg Gly Gln Ser Ala His Ser Leu Leu
65                  70                  75                  80
Glu Ile Lys Ala Val Ala Leu Arg Thr Val Ala Ile Lys Gly Val His
                85                  90                  95
Ser Val Arg Tyr Leu Cys Met Gly Ala Asp Gly Lys Met Gln Gly Leu
            100                 105                 110
Leu Gln Tyr Ser Glu Glu Asp Cys Ala Phe Glu Glu Glu Ile Arg Pro
        115                 120                 125
Asp Gly Tyr Asn Val Tyr Arg Ser Glu Lys His Arg Leu Pro Val Ser
    130                 135                 140
Leu Ser Ser Ala Lys Gln Arg Gln Leu Tyr Lys Asn Arg Gly Phe Leu
145                 150                 155                 160
Pro Leu Ser His Phe Leu Pro Met Leu Pro Met Val Pro Glu Glu Pro
                165                 170                 175
Glu Asp Leu Arg Gly His Leu Glu Ser Asp Met Phe Ser Ser Pro Leu
            180                 185                 190
Glu Thr Asp Ser Met Asp Pro Phe Gly Leu Val Thr Gly Leu Glu Ala
        195                 200                 205
Val Arg Ser Pro Ser Phe Glu Lys
    210                 215

<210> 15
<211> 209
<212> PRT
<213> Homo sapiens

<400> 15
Met Asp Ser Asp Glu Thr Gly Phe Glu His Ser Gly Leu Trp Val Ser
1               5                   10                  15
Val Leu Ala Gly Leu Leu Leu Gly Ala Cys Gln Ala His Pro Ile Pro
            20                  25                  30
Asp Ser Ser Pro Leu Leu Gln Phe Gly Gly Gln Val Arg Gln Arg Tyr
        35                  40                  45
Leu Tyr Thr Asp Asp Ala Gln Gln Thr Glu Ala His Leu Glu Ile Arg
    50                  55                  60
Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95
Lys Thr Ser Arg Phe Leu Cys Gln Arg Pro Asp Gly Ala Leu Tyr Gly
            100                 105                 110
Ser Leu His Phe Asp Pro Glu Ala Cys Ser Phe Arg Glu Leu Leu Leu
        115                 120                 125
Glu Asp Gly Tyr Asn Val Tyr Gln Ser Glu Ala His Gly Leu Pro Leu
    130                 135                 140
His Leu Pro Gly Asn Lys Ser Pro His Arg Asp Pro Ala Pro Arg Gly
145                 150                 155                 160
Pro Ala Arg Phe Leu Pro Leu Pro Gly Leu Pro Pro Ala Leu Pro Glu
                165                 170                 175
Pro Pro Gly Ile Leu Ala Pro Gln Pro Pro Asp Val Gly Ser Ser Asp
            180                 185                 190
Pro Leu Ser Met Val Gly Pro Ser Gln Gly Arg Ser Pro Ser Tyr Ala
        195                 200                 205
Ser

<210> 16
<211> 10
<212> PRT
<213> Artificial Sequence

<220>
<223> Residues which can be incorporated to allow myc
      monoclonal antibody-based affinity purification.

<400> 16
Glu Gln Lys Leu Ile Ser Glu Glu Asp Leu
 1               5                  10

<210> 17
<211> 5
<212> PRT
<213> Artificial Sequence

<220>
<223> Preferred thrombin cleavage site.

<400> 17
Leu Val Pro Arg Gly
 1               5

<210> 18
<211> 10
<212> PRT
<213> Artificial Sequence

<220>
<223> Residues that bind to paramagentic steptavidin
      beads which facilitates purification of molecules.

<400> 18
Ser Ala Trp Arg His Pro Gln Phe Gly Gly
 1               5                  10

<210> 19
<211> 14
<212> PRT

<213> Artificial Sequence

<220>
<223> Oligopeptide used for the production of an
      antibody to FGF-23 protein.  (residues 175-189 of
      SEQ ID NO:4)

<400> 19
Arg Arg His Thr Arg Ser Ala Glu Asp Asp Ser Glu Arg Asp
 1               5                   10

<210> 20
<211> 17
<212> PRT
<213> Artificial Sequence

<220>
<223> Oligopeptide used for the production of an
      antibody to FGF-23 protein.  (residues 51-67 of
      SEQ ID NO:4)

<400> 20
Tyr His Leu Gln Ile His Lys Asn Gly His Val Asp Gly Ala Pro His
 1               5                   10                  15
Gln

<210> 21
<211> 13
<212> PRT
<213> Artificial Sequence

<220>
<223> E tag

<400> 21
Gly Ala Pro Val Pro Tyr Pro Asp Pro Leu Glu Pro Arg
 1               5                   10

<210> 22
<211> 6
<212> PRT
<213> Artificial Sequence

<220>
<223> His6 tag

<400> 22
His His His His His His
 1               5

<210> 23
<211> 111
<212> PRT
<213> Homo sapiens

<400> 23
Ala Lys Arg Ala Phe Leu Pro Gly Met Asn Pro Pro Pro Tyr Ser Gln
 1               5                   10                  15
Phe Leu Ser Arg Arg Asn Glu Ile Pro Leu Ile His Phe Asn Thr Pro

                20                  25                  30
Ile Pro Arg Arg His Thr Arg Ser Ala Glu Asp Asp Ser Glu Arg Asp
        35                  40                  45
Pro Leu Asn Val Leu Lys Pro Arg Ala Arg Met Thr Pro Ala Pro Ala
    50                  55                  60
Ser Cys Ser Gln Glu Leu Pro Ser Ala Glu Asp Asn Ser Pro Met Ala
65                  70                  75                  80
Ser Asp Pro Leu Gly Val Val Arg Gly Gly Arg Val Asn Thr His Ala
                85                  90                  95
Gly Gly Thr Gly Pro Glu Gly Cys Arg Pro Phe Ala Lys Phe Ile
            100                 105                 110

<210> 24
<211> 35
<212> PRT
<213> Homo sapiens

<400> 24
Asn Trp Phe Val Gly Leu Lys Lys Asn Gly Ser Cys Lys Arg Gly Pro
 1               5                  10                  15
Arg Thr His Tyr Gly Gln Lys Ala Ile Leu Phe Leu Pro Leu Pro Val
            20                  25                  30
Ser Ser Asp
        35

<210> 25
<211> 34
<212> PRT
<213> Homo sapiens

<400> 25
Gln Met Tyr Val Ala Leu Asn Gly Lys Gly Ala Pro Arg Arg Gly Gln
 1               5                  10                  15
Lys Thr Arg Arg Lys Asn Thr Ser Ala His Phe Leu Pro Met Val Val
            20                  25                  30
His Ser

<210> 26
<211> 56
<212> PRT
<213> Homo sapiens

<400> 26
Ala Trp Tyr Leu Gly Leu Asp Lys Glu Gly Gln Val Met Lys Gly Asn
 1               5                  10                  15
Arg Val Lys Lys Thr Lys Ala Ala Ala His Phe Leu Pro Lys Leu Leu
            20                  25                  30
Glu Val Ala Met Tyr Gln Glu Pro Ser Leu His Ser Val Pro Glu Ala
        35                  40                  45
Ser Pro Ser Ser Pro Pro Ala Pro
    50                  55

<210> 27
<211> 72
<212> PRT
<213> Homo sapiens

<400> 27
Ala Trp Phe Leu Gly Leu Asn Lys Glu Gly Gln Ile Met Lys Gly Asn

 1               5                   10                  15
Arg Val Lys Lys Thr Lys Pro Ser Ser His Phe Val Pro Lys Pro Ile
            20                  25                  30
Glu Val Cys Met Tyr Arg Glu Pro Ser Leu His Glu Ile Gly Glu Lys
        35                  40                  45
Gln Gly Arg Ser Arg Lys Ser Ser Gly Thr Pro Thr Met Asn Gly Gly
    50                  55                  60
Lys Val Val Asn Gln Asp Ser Thr
65                  70

<210> 28
<211> 78
<212> PRT
<213> Homo sapiens

<400> 28
Gly Trp Tyr Leu Gly Leu Asn Lys Glu Gly Glu Ile Met Lys Gly Asn
 1               5                   10                  15
His Val Lys Lys Asn Lys Pro Ala Ala His Phe Leu Pro Lys Pro Leu
            20                  25                  30
Lys Val Ala Met Tyr Lys Glu Pro Ser Leu His Asp Leu Thr Glu Phe
        35                  40                  45
Ser Arg Ser Gly Ser Gly Thr Pro Thr Lys Ser Arg Ser Val Ser Gly
    50                  55                  60
Val Leu Asn Gly Gly Lys Ser Met Ser His Asn Glu Ser Thr
65                  70                  75

<210> 29
<211> 78
<212> PRT
<213> Homo sapiens

<400> 29
Ala Trp Phe Leu Gly Leu Asn Lys Glu Gly Gln Ala Met Lys Gly Asn
 1               5                   10                  15
Arg Val Lys Lys Thr Lys Pro Ala Ala His Phe Leu Pro Lys Pro Leu
            20                  25                  30
Glu Val Ala Met Tyr Arg Glu Pro Ser Leu His Asp Val Gly Glu Thr
        35                  40                  45
Val Pro Lys Pro Gly Val Thr Pro Ser Lys Ser Thr Ser Ala Ser Ala
    50                  55                  60
Ile Met Asn Gly Gly Lys Pro Val Asn Lys Ser Lys Thr Thr
65                  70                  75

<210> 30
<211> 78
<212> PRT
<213> Homo sapiens

<400> 30
Ala Trp Phe Leu Gly Leu Asn Lys Glu Gly Gln Ala Met Lys Gly Asn
 1               5                   10                  15
Arg Val Lys Lys Thr Lys Pro Ala Ala His Phe Leu Pro Lys Pro Leu
            20                  25                  30
Glu Val Ala Met Tyr Arg Glu Pro Ser Leu His Asp Val Gly Glu Thr
        35                  40                  45
Val Pro Lys Pro Gly Val Thr Pro Ser Lys Ser Thr Ser Ala Ser Ala
    50                  55                  60
Ile Met Asn Gly Gly Lys Pro Val Asn Lys Ser Lys Thr Thr
65                  70                  75

<210> 31
<211> 48
<212> PRT
<213> Homo sapiens

<400> 31
Gln Tyr Tyr Val Ala Leu Asn Lys Asp Gly Ser Pro Arg Glu Gly Tyr
1               5                   10                  15
Arg Thr Lys Arg His Gln Lys Phe Thr His Phe Leu Pro Arg Pro Val
            20                  25                  30
Asp Pro Ser Lys Leu Pro Ser Met Ser Arg Asp Leu Phe His Tyr Arg
        35                  40                  45

<210> 32
<211> 68
<212> PRT
<213> Homo sapiens

<400> 32
Trp Phe Met Ala Phe Thr Arg Gln Gly Arg Pro Arg Gln Ala Ser Arg
1               5                   10                  15
Ser Arg Gln Asn Gln Arg Glu Ala His Phe Ile Lys Arg Leu Tyr Gln
            20                  25                  30
Gly Gln Leu Pro Phe Pro Asn His Ala Glu Lys Gln Lys Gln Phe Glu
        35                  40                  45
Phe Val Gly Ser Ala Pro Thr Arg Arg Thr Lys Arg Thr Arg Arg Pro
    50                  55                  60
Gln Pro Leu Thr
65

<210> 33
<211> 59
<212> PRT
<213> Homo sapiens

<400> 33
Trp Tyr Val Gly Phe Thr Lys Lys Gly Arg Pro Arg Lys Gly Pro Lys
1               5                   10                  15
Thr Arg Glu Asn Gln Gln Asp Val His Phe Met Lys Arg Tyr Pro Lys
            20                  25                  30
Gly Gln Pro Glu Leu Gln Lys Pro Phe Lys Tyr Thr Thr Val Thr Lys
        35                  40                  45
Arg Ser Arg Arg Ile Arg Pro Thr His Pro Ala
    50                  55

<210> 34
<211> 76
<212> PRT
<213> Homo sapiens

<400> 34
Leu Pro Val Ser Leu Ser Ser Ala Lys Gln Arg Gln Leu Tyr Lys Asn
1               5                   10                  15
Arg Gly Phe Leu Pro Leu Ser His Phe Leu Pro Met Leu Pro Met Val
            20                  25                  30
Pro Glu Glu Pro Glu Asp Leu Arg Gly His Leu Glu Ser Asp Met Phe
        35                  40                  45
Ser Ser Pro Leu Glu Thr Asp Ser Met Asp Pro Phe Gly Leu Val Thr
    50                  55                  60

Gly Leu Glu Ala Val Arg Ser Pro Ser Phe Glu Lys
65                  70                  75

<210> 35
<211> 33
<212> PRT
<213> Homo sapiens

<400> 35
Trp Tyr Val Ala Leu Lys Arg Thr Gly Gln Tyr Lys Leu Gly Ser Lys
 1               5                  10                  15
Thr Gly Pro Gly Gln Lys Ala Ile Leu Phe Leu Pro Met Ser Ala Lys
            20                  25                  30
Ser

<210> 36
<211> 68
<212> PRT
<213> Homo sapiens

<400> 36
Leu Pro Leu His Leu Pro Gly Asn Lys Ser Pro His Arg Asp Pro Ala
 1               5                  10                  15
Pro Arg Gly Pro Ala Arg Phe Leu Pro Leu Pro Gly Leu Pro Pro Ala
            20                  25                  30
Leu Pro Glu Pro Pro Gly Ile Leu Ala Pro Gln Pro Pro Asp Val Gly
        35                  40                  45
Ser Ser Asp Pro Leu Ser Met Val Gly Pro Ser Gln Gly Arg Ser Pro
    50                  55                  60
Ser Tyr Ala Ser
65

<210> 37
<211> 88
<212> PRT
<213> Homo sapiens

<400> 37
Leu Trp Tyr Val Ser Val Asn Gly Lys Gly Arg Pro Arg Arg Gly Phe
 1               5                  10                  15
Lys Thr Arg Arg Thr Gln Lys Ser Ser Leu Phe Leu Pro Arg Val Leu
            20                  25                  30
Asp His Arg Asp His Glu Met Val Arg Gln Leu Gln Ser Gly Leu Pro
        35                  40                  45
Arg Pro Pro Gly Lys Gly Val Gln Pro Arg Arg Arg Arg Gln Lys Gln
    50                  55                  60
Ser Pro Asp Asn Leu Glu Pro Ser His Val Gln Ala Ser Arg Leu Gly
65                  70                  75                  80
Ser Gln Leu Glu Ala Ser Ala His
                85

<210> 38
<211> 30
<212> PRT
<213> Homo sapiens

<400> 38
Met Phe Ile Ala Leu Ser Lys Asn Gly Lys Thr Lys Lys Gly Asn Arg
 1               5                  10                  15

Val Ser Pro Thr Met Lys Val Thr His Phe Leu Pro Arg Leu
            20                  25                  30

<210> 39
<211> 82
<212> PRT
<213> Homo sapiens

<400> 39
Glu Trp Tyr Val Ala Leu Asn Lys Arg Gly Lys Ala Lys Arg Gly Cys
 1               5                  10                  15
Ser Pro Arg Val Lys Pro Gln His Ile Ser Thr His Phe Leu Pro Arg
            20                  25                  30
Phe Lys Gln Ser Glu Gln Pro Glu Leu Ser Phe Thr Val Thr Val Pro
        35                  40                  45
Glu Lys Lys Lys Pro Pro Ser Pro Ile Lys Pro Lys Ile Pro Leu Ser
    50                  55                  60
Ala Pro Arg Lys Asn Thr Asn Ser Val Lys Tyr Arg Leu Lys Phe Arg
65                  70                  75                  80
Phe Gly

<210> 40
<211> 30
<212> PRT
<213> Homo sapiens

<400> 40
Thr Tyr Ile Ala Leu Ser Lys Tyr Gly Arg Val Lys Arg Gly Ser Lys
 1               5                  10                  15
Val Ser Pro Ile Met Thr Val Thr His Phe Leu Pro Arg Ile
            20                  25                  30

<210> 41
<211> 33
<212> PRT
<213> Homo sapiens

<400> 41
Glu Met Phe Val Ala Leu Asn Gln Lys Gly Ile Pro Val Arg Gly Lys
 1               5                  10                  15
Lys Thr Lys Lys Glu Gln Lys Thr Ala His Phe Leu Pro Met Ala Ile
            20                  25                  30
Thr

<210> 42
<211> 67
<212> PRT
<213> Homo sapiens

<400> 42
Trp Tyr Met Ala Phe Thr Arg Lys Gly Arg Pro Arg Lys Gly Ser Lys
 1               5                  10                  15
Thr Arg Gln His Gln Arg Glu Val His Phe Met Lys Arg Leu Pro Arg
            20                  25                  30
Gly His His Thr Thr Glu Gln Ser Leu Arg Phe Glu Phe Leu Asn Tyr
        35                  40                  45
Pro Pro Phe Thr Arg Ser Leu Arg Gly Ser Gln Arg Thr Trp Ala Pro
    50                  55                  60

Glu Pro Arg
65

<210> 43
<211> 48
<212> PRT
<213> Homo sapiens

<400> 43
Arg Tyr Tyr Val Ala Leu Asn Lys Asp Gly Thr Pro Arg Glu Gly Thr
1               5                   10                  15
Arg Thr Lys Arg His Gln Lys Phe Thr His Phe Leu Pro Arg Pro Val
            20                  25                  30
Asp Pro Asp Lys Val Pro Glu Leu Tyr Lys Asp Ile Leu Ser Gln Ser
        35                  40                  45

<210> 44
<211> 48
<212> PRT
<213> Homo sapiens

<400> 44
Arg Tyr Phe Val Ala Leu Asn Lys Asp Gly Thr Pro Arg Asp Gly Ala
1               5                   10                  15
Arg Ser Lys Arg His Gln Lys Phe Thr His Phe Leu Pro Arg Pro Val
            20                  25                  30
Asp Pro Glu Arg Val Pro Glu Leu Tyr Lys Asp Leu Leu Met Tyr Thr
        35                  40                  45

<210> 45
<211> 32
<212> PRT
<213> Homo sapiens

<400> 45
Met Phe Leu Ala Leu Asp Arg Arg Gly Gly Pro Arg Pro Gly Gly Arg
1               5                   10                  15
Thr Arg Arg Tyr His Leu Ser Ala His Phe Leu Pro Val Leu Val Ser
            20                  25                  30

<210> 46
<211> 22
<212> PRT
<213> Artificial Sequence

<220>
<223> consensus sequence

<400> 46
Trp Tyr Val Ala Leu Lys Gly Pro Arg Lys Gly Arg Thr Lys Lys Ala
1               5                   10                  15
His Phe Leu Pro Arg Val
            20

<210> 47
<211> 9
<212> PRT
<213> Artificial Sequence

<220>

<223> FGF-23 cleavage products

<400> 47
Asn Ala Ser Pro Leu Leu Gly Ser Ser
 1               5

<210> 48
<211> 10
<212> PRT
<213> Artificial Sequence

<220>
<223> FGF-23 cleavage products

<220>
<221> VARIANT
<222> (1)...(2)
<223> Xaa = any amino acid

<400> 48
Xaa Xaa Trp Gly Gly Leu Ile His Leu Tyr
 1               5                   10

<210> 49
<211> 10
<212> PRT
<213> Artificial Sequence

<220>
<223> FGF-23 cleavage products

<400> 49
Ser Ala Glu Asp Asp Ser Glu Arg Asp Pro
 1               5                   10